# EUROPÄISCHE PATENTSCHRIFT

(11) **EP 3 145 978 B1**
(45) Veröffentlichungstag und Bekanntmachung des Hinweises auf die Patenterteilung: **27.06.2018**
(21) Anmeldenummer: 15719679.1
(22) Anmeldetag: 28.04.2015
(51) Int. Cl.: C08G 65/26, B01J 27/26, C08G 77/46

(54) **HOCH AKTIVE DOPPELMETALLCYANID-KATALYSATOREN UND VERFAHREN ZU DEREN HERSTELLUNG**
HIGHLY ACTIVE DOUBLE METAL CYANIDE CATALYSTS AND METHOD FOR THE PRODUCTION THEREOF
CATALYSEURS À BASE DE CYANURE BIMÉTALLIQUE HAUTEMENT ACTIFS ET PROCÉDÉ DE PRODUCTION

(30) Priorität: 19.05.2014 DE 102014209407
(43) Veröffentlichungstag der Anmeldung: 29.03.2017
(73) Patentinhaber: Evonik Degussa GmbH, 45128 Essen (DE)
(72) Erfinder: FIEDEL, Olga, 45131 Essen (DE); SCHUBERT, Frank, 47506 Neukirchen-Vluyn (DE); FIEDEL, Michael, 45131 Essen (DE); KNOTT, Wilfried, 45355 Essen (DE)
(86) Internationale Anmeldenummer: PCT/EP2015/059120
(87) Internationale Veröffentlichungsnummer: WO 2015/176920

(56) Entgegenhaltungen:
- EP-A1- 0 485 637
- WO-A1-01/90219
- WO-A1-99/64152
- US-A1- 2010 105 843
- US-A1- 2010 168 367
- US-B1- 6 362 126

## Beschreibung

### Gegenstand der Erfindung:

Die Erfindung betrifft DMC-Katalysatoren enthaltend Polyethersiloxane, Verfahren zu deren Herstellung, deren Verwendung so wie die durch die erfindungsgemäßen Verfahren erhältlichen DMC-Katalysatoren.

### Stand der Technik:

Im Stand der Technik sind bereits unterschiedliche Verfahren zur Herstellung von Doppelmetallcyanid Katalysatoren (im Folgenden DMC Katalysatoren) bekannt, in welchen oberflächenaktive Substanzen eingesetzt werden.

Die WO 2000/74843 A1 und die WO 2000/74845 A1 offenbaren Verfahren zur Herstellung von plättchenförmigen Multimetallcyanid-Verbindungen und deren Verwendung zur Herstellung von Polyetherpolyolen. Zur Herstellung solcher plättchenförmiger Multimetallcyanid-Verbindungen können den Metallsalzen oder den Cyanometallverbindungen oberflächenaktive Komponenten, wie beispielsweise Umsetzungsprodukte aus Fettalkoholen mit Alkylenoxiden, erhältlich unter den Markennamen Pluronic® oder Plurafac® (Produkte der Firma BASF SE), zugefügt werden. Gemäß der Lehre der WO 2000/74843 und der WO 2000/74845 können so Multimetallcyanid-Verbindungen mit einer veränderten Morphologie hergestellt werden. Diese plättchenförmige Morphologie führt angeblich dazu, dass der Anteil an katalytisch aktiver Oberfläche, bezogen auf die Gesamtoberfläche, zunimmt und damit die massenspezifische Aktivität steigt. Auffällig ist, dass die Beispiele zur Herstellung von Polyethern mit den beschriebenen DMC-Katalysatoren ausschließlich den Reaktionsstart mit Propylenoxid zeigen.

Gegenstand der WO 2004/000913 A1 ist ein Verfahren zur Herstellung von Polyetheralkoholen unter Verwendung von DMC-Katalysatoren, wobei die DMC-Katalysatoren unter ganz bestimmten Reaktionsbedingungen, beispielsweise bezüglich Temperatur und Rührleistung, hergestellt werden. Die DMC-Katalysatoren, die bei diesem speziellen Verfahren zum Einsatz kommen können einen organischen Zusatzstoff enthalten, welcher aus einer Liste bekannter Polymere, bevorzugt aus Fettalkoholen mit Alkylenoxiden, erhältlich unter den Markennamen Pluronic® oder Plurafac® (Produkte der Firma BASF SE), auszuwählen ist. Auch in diesem Fall zeigen die Beispiele augenscheinlich nur solche Verfahren zur Herstellung von Polyethern mit den beschriebenen DMC-Katalysatoren, bei welchen der Reaktionsstart mit Propylenoxid erfolgt.

EP1256596 A1 offenbart Verfahren zur Herstellung von DMC-Katalysatoren, die mindestens eine oberflächenaktive Substanz enthalten. Gleichermaßen befassen sich die WO 00/47650 A1 und die EP1165658 A1 mit DMC-Katalysatoren, die mindestens eine oberflächenaktive Substanz enthalten. Auch in diesen Fällen wird anhand der erhaltenen DMC-Katalysatoren ausschließlich die Umsetzung von Propylenoxid gezeigt.

DMC-Katalysatoren werden bekanntermaßen neben weiteren Reaktionen insbesondere zur Alkoxylierung von Epoxiden eingesetzt. Dabei werden immer wieder die bekannten Epoxide Ethylenoxid, Propylenoxid und Butylenoxid und weitere angeführt. Während der Start der Reaktion wie allgemein bekannt mit Propylenoxid und anderen Epoxiden höherer Molmassen problemlos durchführbar ist, gibt es jedoch nur wenige experimentelle Beispiele, in welchen eine DMCkatalysierte Alkoxylierung mit reinem Ethylenoxid gestartet wird, oder reine Ethylenoxid basierte Polyether mittels DMC Katalyse hergestellt werden. Auch die Beispiele der vorab beschriebenen Dokumente zeigen den Start des DMC-Katalysators mit Propylenoxid. Dies liegt daran, dass Ethylenoxide an der überwiegenden Anzahl der literaturbekannten DMC-Katalysatoren als Katalysatorgift wirken, das heißt den Katalysator blockieren, und damit die Aktivität des Katalysators drastisch abfällt bzw. ganz zum Erliegen kommt. Daher ist es in der Praxis üblich, die Katalysatoren zunächst mit einem gut verträglichen Epoxid, beispielsweise Propylenoxid zu starten und erst im weiteren Reaktionsverlauf Ethylenoxid zuzugeben.

Es herrscht daher nach wie vor Bedarf an Katalysatoren, welche weniger sensibel auf kleinere Reaktanden, wie beispielsweise Ethylenoxid, reagieren und die bevorzugt gleichzeitig gute Reaktionskinetiken und kurze Induktionsperioden aufweisen.

Aufgabe der vorliegenden Erfindung war es daher, Katalysatoren bereit zu stellen, welche in Gegenwart von Ethylenoxid als einzigem Epoxid gestartet werden können. Weitere Aufgabe der Erfindung war es, Katalysatoren bereit zu stellen, die eine schnelle Reaktionskinetik, eine vergleichsweise kurze Induktionsperiode aufweisen und die, durch ihre Eigenschaften in der Lage sind, das Spektrum an bislang über DMC-Katalyse herstellbaren Alkoxylierungsprodukten deutlich zu erweitern.

### Detaillierte Beschreibung:

Im Rahmen dieser Erfindung wurde nun gefunden, dass DMC-Katalysatoren, enthaltend Polyethersiloxane, wie in den vorliegenden Ansprüchen beschrieben, die gestellten Aufgaben hervorragend lösen.

Die erfindungsgemäßen Katalysatoren, das Verfahren zu deren Herstellung sowie deren Verwendung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Werden nachfolgend Angaben in Prozent gemacht, so handelt es sich, wenn nicht anders angegeben um Angaben in Gewichts-%. Werden nachfolgend Mittelwerte angegeben, so handelt es sich, wenn nicht anders angegeben, um das Zahlenmittel. Werden nachfolgend Stoffeigenschaften, wie z. B. Viskositäten oder ähnliches angegeben, so handelt es sich, wenn nicht anders angegeben, um die Stoffeigenschaften gemessen bei 25 °C. Die Viskosität wird bei einer Temperatur von 25 °C mit einer Scherrate von 10 1/s mit einem MCR 301 Rheometer der Firma Anton Paar bestimmt.

Gegenstand der vorliegenden Erfindung sind daher DMC-Katalysatoren, wie in den vorliegenden Ansprüchen beschrieben, die bevorzugt über das erfindungsgemäße Verfahren erhältlich sind, enthaltend
a) ein oder mehrere Doppelmetallcyanid-Verbindungen und
b) ein oder mehrere Polyethersiloxane und
c) optional einen oder mehrere von b) verschiedenen organischen Komplexliganden.

Bei Komponente a) handelt es sich um Doppelmetallcyanid-Verbindungen, die Reaktionsprodukte wasserlöslicher Metallsalze der Komponente a1) und wasserlöslicher Metallcyanidsalze der Komponente a2) sind.

Zur Herstellung der Doppelmetallcyanid-Verbindungen geeignete wasserlösliche Metallsalze der Komponente a1) besitzen bevorzugt die allgemeine Formel (I)

M(X)ₙ Formel (I),

wobei M ausgewählt wird aus den Metallen Zn (II), Fe (II), Ni (II), Mn (II), Co (II), Sn (II), Pb (II), Fe (III), Mo (IV), Mo(VI), Al (III), V (V), V (IV), Sr (II), W (IV), W (VI), Cu (II) und Cr (III). Besonders bevorzugt sind Zn (II), Fe (II), Co (II) und Ni (II). X sind gleiche oder verschiedene, vorzugsweise gleiche Anionen, bevorzugt ausgewählt aus der Gruppe der Halogenide, Hydroxide, Sulfate, Carbonate, Cyanate, Thiocyanate, Isocyanate, Isothiocyanate, Carboxylate, Oxalate oder Nitrate. Der Wert für n ist 1, 2 oder 3. Beispiele geeigneter wasserlöslicher Metallsalze sind Zinkchlorid, Zinkbromid, Zinkacetat, Zinkacetylacetonat, Zinkbenzoat, Zinknitrat, Eisen(II)sulfat, Eisen(II)bromid, Eisen(II)chlorid, Cobalt(II)chlorid, Cobalt(II)thiocyanat, Nickel(II)chlorid und Nickel(II)nitrat. Es können auch Mischungen verschiedener wasserlöslicher Metallsalze eingesetzt werden.

Zur Herstellung der Doppelmetallcyanid-Verbindungen geeignete wasserlösliche Metallcyanidsalze der Komponente a2) besitzen bevorzugt die allgemeine Formel (II)

(Y)ₐM'(CN)_{b}(A)_{c} (II)

wobei M' ausgewählt wird aus den Metallen Fe(II), Fe(III), Co(II), Co(III), Cr(II), Cr(III), Mn(II), Mn(III), Ir(III), Ni(II), Rh (III), Ru(II), V(IV) und V(V). Besonders bevorzugt wird M' ausgewählt aus den Metallen Co(II), Co(III), Fe(II), Fe(III), Cr(III), Ir(III) und Ni(II). Das wasserlösliche Metallcyanidsalz kann eines oder mehrere dieser Metalle enthalten. Y sind gleiche oder verschiedene, vorzugsweise gleiche Alkalimetallkationen oder Erdalkalimetallkationen. A sind gleiche oder verschiedene, vorzugsweise gleiche Anionen, ausgewählt aus der Gruppe der Halogenide, Hydroxide, Sulfate, Carbonate, Cyanate, Thiocyanate, Isocyanate, Isothiocyanate, Carboxylate, Oxalate oder Nitrate. Sowohl a, als auch b und c sind ganzzahlig, wobei die Werte für a, b und c so gewählt sind, dass die Elektroneutralität des Metallcyanidsalzes gegeben ist; a ist vorzugsweise 1, 2, 3 oder 4; b ist vorzugsweise 4, 5 oder 6; c besitzt bevorzugt den Wert 0.

Beispiele geeigneter wasserlöslicher Metallcyanidsalze sind Kaliumhexacyanocobaltat(III), Kaliumhexacyanoferrat(II), Kaliumhexacyanoferrat(III), Calciumhexacyanocobaltat(III) und Lithiumhexacyanocobaltat(III).

Bevorzugte Doppelmetallcyanid-Verbindungen der Komponente a) sind Verbindungen der allgemeinen Formel (III)

Mₓ[M'_{x'}(CN)_{y}]_{z} (III)

worin M wie in Formel (I) und M' wie in Formel (II) definiert ist, und x, x', y und z ganzzahlig und so gewählt sind, dass die Elektroneutralität der Doppelmetallcyanidverbindung gegeben ist. Vorzugsweise ist x 3, x' 1, Y 6 und z 2, M Zn(II), Fe(II), Co(II) oder Ni(II) und M' Co(III), Fe(III), Cr(III) oder Ir(III).

Beispiele geeigneter Doppelmetallcyanidverbindungen der Komponente a) sind Zinkhexacyanocobaltat(III), Zinkhexacyanoiridat (III), Zinkhexacyanoferrat(III) und Cobalt(II)hexacyanocobaltat(III). Weitere Beispiele geeigneter Doppelmetallcyanid Verbindungen sind z.B. US-A 5 158922 zu entnehmen. Besonders bevorzugt verwendet wird Zinkhexacyanocobaltat (III).

Als Polyethersiloxan der Komponente b) kann prinzipiell jedes Polyethersiloxan verwendet werden. Polyethersiloxane im Sinne dieser Erfindung sind alle Verbindungen, die sowohl einen Siloxan-Anteil als auch einen Polyether-Anteil enthalten. Wie sich herausgestellt hat sind DMC-Katalysatoren enthaltend ein Polyethersiloxan der Komponente b) deutlich reaktiver, weisen schnelle Induktionszeiten auf und sind zudem in der Lage reine Ethylenoxid-Polyether aufzubauen, ohne dabei nennenswert an Aktivität einzubüßen. Es hat sich gezeigt, dass insbesondere solche erfindungsgemäßen DMC-Katalysatoren besonders schnelle Induktionszeiten aufweisen, bei denen die berechnete mittlere Molmasse des Polyethersiloxans von 300 bis 50.000 g/Mol, bevorzugt von 500 bis 30.000 g/Mol , besonders bevorzugt von 600 bis 10.000 g/Mol beträgt und/oder, bevorzugt und, das Polyethersiloxan ein Gewichtsverhältnis von Siloxan- Anteil zu Polyether-Anteil von 1 zu 1 bis 1 zu 10, bevorzugt 1 zu 1,2 bis 1 zu 8, besonders bevorzugt von 1 zu 1,5 bis 1 zu 5, und insbesondere bevorzugt von 1 zu 1,8 bis 1 zu 3, bezogen auf die berechnete mittlere Molmasse des Polyethersiloxans aufweist. In anderen Worten beträgt der Quotient der gebildet wird aus der berechneten mittleren Molmasse von Siloxan-Anteil geteilt durch berechnete mittlere Molmasse von Polyether-Anteil von 0,1 bis 1 bevorzugt von 0,2 bis 0,9, besonders bevorzugt von 0,4 bis 0,6.

Die berechnete mittlere Molmasse wird im Sinne dieser Erfindung wie folgt bestimmt: Von dem eingesetzten Polyethersiloxan wird sowohl ein ²⁹Si-NMR als auch ein ¹H-NMR gemessen. Aus dem ²⁹Si-NMR ergeben sich die Anteile an M, D, T und Q Einheiten. Aus dem ²⁹Si-NMR ergibt sich ebenso, ob und welche Anzahl an M und D Einheiten am Siloxan mit Polyether oder anderen Gruppen substituiert sind. Aus den so erhaltenen Angaben wird die Molmasse des Siloxan-Anteils berechnet. Analog werden aus dem ¹H-NMR die Anteile der unterschiedlichen Alkylenoxide im Polyether-Anteil bestimmt und die Anteile der Starter. Aus den so erhaltenen Angaben wird die Molmasse des Polyether-Anteils berechnet. Aus beiden Anteilen ergibt sich die berechnete mittlere Molmasse des Polyethersiloxans. ²⁹Si-NMR und ¹H-NMR werden in CDCl₃ als Lösungsmittel gemessen. Alternativ, insbesondere für den Fall, dass einzelne Polyethersiloxane zu komplex sein sollten, um ihre mittlere Molmasse durch die oben beschriebene Methode zu berechnen, kann auch vor der Herstellung des Polyethersiloxans der Polyether vor der Reaktion mit dem Siloxan mit ¹H-NMR bestimmt und seine mittlere Molmasse berechnet werden und das Siloxan vor der Reaktion mit dem Polyether mit ²⁹Si-NMR bestimmt und seine mittlere Molmasse berechnet werden. Aus den berechneten mittleren Molmassen der beiden Ausgangsstoffe Polyether und Siloxan kann der Fachmann die mittlere Molmasse des Produktes Polyethersiloxan berechnen.

Zur Berechnung des Gewichtsverhältnisses werden dabei alle Polyether-Reste bis zur Bindungsstelle an das Si-Atom (R² in Formel IV) vollständig dem Polyether-Anteil zugerechnet und das Siloxangerüst mit allen weiteren Substituenten dem Siloxan-Anteil zugerechnet. Polyethersiloxane mit den beschriebenen Gewichtsverhältnissen zwischen Polyether-Anteil und Siloxan-Anteil erzielen ganz besonders hervorragende DMC-Katalysatoren im Vergleich zu den im Stand der Technik bekannten modifizierten oder mit anderen als der hier definierten Komponente b) modifizierten DMC Katalysatoren.

Besonders bevorzugt sind Polyethersiloxane welche Polydialkylsiloxane, insbesondere Polydimethylsiloxane, umfassen, die 1 bis 100, bevorzugt 1 bis 60 D-Siloxaneinheiten aufweisen und bei denen an einer Position die Alkylgruppe, insbesondere die Methylgruppe, durch einen Polyether mit 2 bis 50, bevorzugt 3 bis 20 Alkylenoxid-Einheiten, bevorzugt Ethylenoxid-Einheiten ausgetauscht ist. Zudem ist bevorzugt, wenn der Polyether am freien Ende (also nicht dem Ende, an dem die Bindung an das Siloxan erfolgt ist) eine OH-Gruppe trägt.

Es kann auch besonders vorteilhaft sein, wenn neben dem Polyethersiloxan ein Anteil, bevorzugt größer 0 bis 2 Mol Äquivalente, bezogen auf das Polyethersiloxan, an reinem Polyether anwesend ist, der in seinem Aufbau dem Polyether des Polyethersiloxans im Wesentlichen entspricht.

Bevorzugte Polyethersiloxane der Komponente b) entsprechen der Formel (IV)

M_{d} M'_{d1} Dₑ D'ₑ₁ D"ₑ₂ T_{f} Q_{g} (Formel IV)

wobei
- M: = (R'₃Si O_{1/2})
- M': = (R²R¹₂Si O_{1/2})
- D: = (R¹₂ Si O_{2/2})
- D': = (R²R¹ Si O_{2/2})
- D": = (R⁴R¹ Si O_{2/2})
- T: = (R³ Si O_{3/2})
- Q: = (Si O_{4/2})

- d: = 0 bis 20; bevorzugt 1 bis 10, besonders bevorzugt 1 bis 5 und insbesondere bevorzugt 2;
- d1: = 0 bis 20; bevorzugt 1 bis 10, besonders bevorzugt 0 bis 2; insbesondere bevorzugt 0;
wobei die Summe aus d und d1 bevorzugt 2 ergibt;
- e: = 0 bis 300; bevorzugt 1 bis 100, besonders bevorzugt 2 bis 40, insbesondere bevorzugt 0 bis 20;
- e1: = 0 bis 25; bevorzugt 0,1 bis 15, besonders bevorzugt 1 bis 10, insbesondere bevorzugt 1 bis 5;
- e2: = 0 bis 10; bevorzugt 1 bis 5, insbesondere bevorzugt 0;
- f: = 0 bis 10; bevorzugt 1 bis 5, insbesondere bevorzugt 0;
- g: = 0 bis 10; bevorzugt 1 bis 5, insbesondere bevorzugt 0;
mit der Maßgabe, dass
die Summe aus d1 und e1 größer 0, bevorzugt größer gleich 1 ist;
- R¹: unabhängig voneinander Wasserstoff oder gleiche oder verschiedene lineare oder verzweigte Kohlenwasserstoffreste mit 1 bis 30 Kohlenstoffatomen oder auch aromatische Kohlenwasserstoffreste mit 6 bis 30 Kohlenstoffatomen bevorzugt Methyl oder Phenyl, insbesondere Methyl;
- R²: unabhängig voneinander gleiche oder verschiedene Polyether, wobei die Polyether Seitenketten aufweisen können, die gegebenenfalls auch mit weiteren Heteroatomen substituiert sein können, bevorzugt wird R² ausgewählt aus der Gruppe aus

-(O)ₕ-CH₂-CH₂-CH₂-O-(CH₂-CH₂O-)ⱼ-(CH₂-CH(R')O-)ₖ-(CH(R')-CH₂O-)_{k'}-R" (Formel V)

und

-(O)ₕ-CH₂-CH₂-O-(CH₂-CH₂O-)ⱼ-(CH₂-CH(R')O-)ₖ-(CH(R')-CH₂O-)_{k'}-R" (Formel VI)

-(O)ₕ-(CH₂-CH₂O-)ⱼ-(CH₂-CH(R')O-)ₖ-(CH(R')-CH₂O-)_{k'}- R" (Formel VII)

worin
h 0 oder 1, bevorzugt 0
j 0 bis 100, vorzugsweise größer 0 bis 50, besonders bevorzugt 2 bis 20 insbesondere 3 bis 15,
k + k'= 0 bis 100, vorzugsweise größer 0 bis 50, insbesondere 2 bis 20, insbesondere 0
mit der Maßgabe, dass die Summe aus j, k und k' mindestens 3 ergibt und die Einheiten mit den Indices j, k und k' in zueinander beliebiger Abfolge, also blockweise oder statistisch verteilt, in dem Rest R² angeordnet sind,
   - R': unabhängig von einander eine gegebenenfalls substituierte, beispielsweise mit Alkylresten, Arylresten oder Halogenalkyl- oder Halogenarylresten substituierte, Alkyl- oder Arylgruppe mit 1 bis 12 C-Atomen, bevorzugt Ethyl oder Methyl, insbesondere Methyl, und
   - R": unabhängig voneinander ein Wasserstoffrest oder eine Alkyl- oder Alkylengruppe mit 1 bis 4 C-Atomen, eine Gruppe -C(O)-R''' mit R''' = Alkylrest, eine Gruppe - CH₂C(O)-CH₂C(O)-R', eine Alkylarylgruppe, wie z. B. eine Benzylgruppe, die Gruppe -C(O)NH-R' bedeutet, bevorzugt ist R" ein Wasserstoffrest
   R³ unabhängig voneinander gleiche oder verschiedene Reste R¹ oder R², bevorzugt R¹, besonders bevorzugt Methyl oder Phenyl, insbesondere Methyl.
   R⁴ unabhängig voneinander gleiche oder verschiedene organische Reste mit mehr als 3 Kohlenstoffatomen, bevorzugt mit 4 bis 30, besonders bevorzugt mit 4 bis 20 Kohlenstoffatomen, mit der Maßgabe, dass R⁴ verschieden von R² ist, bevorzugt ist R⁴ ausgewählt aus -CH₂(CH₂)ₙCH₂-O-CH₂(CHOCH₂), wobei (CHOCH₂) einen Epoxidring darstellt, -CH₂(CH₂)ₙC(O)O-CH₃,, -CH₂(CH₂)ₙCH₂OH, - CH₂(CH₂)ₙCH₂O-CH₂CH(OH)CH₂CH₂(OH) mit n = 0 bis 20, bevorzugt 1 bis 10.

Die Polyether können sowohl über eine Si-O-C als auch über eine Si-C Bindung an das Siloxangerüst gebunden sein. Bevorzugt im Sinne dieser Erfindung ist die Si-C Verbindung wie sie beispielsweise als Produkt der Hydrosilylierung erhalten wird. In Formel (IV) stellt der Rest R² mit h = 1 eine Si-O-C Bindung dar und das bevorzugte h = 0 eine Si-C Bindung. Ganz besonders hervorragend eignen sich erfindungsgemäß Polyethersiloxane der Komponente b) gemäß Formel (IV) mit d = 2, d1 = 0, e = 0 bis 40, e1 = 1 bis 5, e2, f und g = 0, R¹ = Methyl, R² = (Formel V), (Formel VI) und/ oder (Formel VII) mit h = 0, j = 3 bis 20, k = 0 bis 20, bevorzugt 0, R' = Methyl oder Ethyl und R" = Wasserstoff. Erfindungsgemäß können alle erhältlichen Polyethersiloxane eingesetzt werden.

DMC-Katalysatoren, die gemäß der vorliegenden Erfindung Polyethersiloxane der Komponente b) enthalten weisen beim Einsatz als Katalysator beispielsweise in Alkoxylierungen von Epoxiden erstaunlich kurze Induktionsperioden auf und zeigen sehr gute Reaktivitäten. Zudem wird ihre Katalysatoraktivität bei der Umsetzung von hohen Anteilen an bzw. reinem Ethylenoxid, insbesondere auch gleich zum Start der Reaktion, nicht negativ beeinflusst. Die Beispiele zeigen hier erstaunliche Fortschritte des erfindungsgemäßen Katalysators beispielsweise im Vergleich zu im Stand der Technik bekannten DMC-Katalysatoren, die mit anderen oberflächenaktiven Substanzen modifiziert wurden, beispielsweise mit Umsetzungsprodukten aus Fettalkoholen mit Alkylenoxiden.

Organische Komplexliganden der Komponente c), die in den erfindungsgemäßen Katalysatoren enthalten sein können, sind wasserlösliche, organische Verbindungen mit Heteroatomen, wie Sauerstoff, Stickstoff, Phosphor oder Schwefel, die mit der Doppelmetallcyanid-Verbindung Komplexe bilden können. Geeignete organische Komplexliganden sind z.B. Alkohole, Aldehyde, Ketone, Ether, Ester, Amide, Harnstoffe, Nitrile, Sulfide und deren Mischungen. Bevorzugte organische Komplexliganden sind wasserlösliche aliphatische Alkohole, wie Ethanol, Isopropanol, n-Butanol, iso-Butanol, sek.-Butanol und tert.-Butanol. Besonders bevorzugt ist tert.-Butanol.

Gewünschtenfalls können die erfindungsgemäßen Katalysatoren als weitere Komponente d) weitere von b) und c) verschiedene komplexbildende Komponenten aufweisen. Bevorzugt handelt es sich bei der komplexbildenden Komponente d) um eine Komponente ausgewählt aus Polyether, Polyester, Polycarbonat, Glycidylether, Glycosid, Carbonsäureester mehrwertiger Alkohole, Polyalkylenglykolsorbitanester, Gallensäure, Salze der Gallensäure, Ester der Gallensäure, Amid der Gallensäure, Cyclodextrin, organisches Phophat, Phosphit, Phosphonat, Phosphonit, Phosphinat oder Phosphinit, eine ionische oberflächen- bzw. grenzflächenaktive Verbindung oder ein α,β-ungesättigter Carbonsäureester. Besonders bevorzugt handelt es sich um Polyether, insbesondere bevorzugt um Polyethylenglycole oder Polypropylenglycole, Polyethylenglycol- und Polypropylenglycolether, Poly(oxyethylen)-co-poly(oxypropylen), Poly(oxyethylen)-co-poly(oxypropylene)ether.

Eine bevorzugte Ausführungsform der vorliegenden Erfindung sind daher DMC-Katalysatoren, die bevorzugt über das erfindungsgemäße Verfahren erhältlich sind, enthaltend
a) ein oder mehrere Doppelmetallcyanid-Verbindungen und
b) ein oder mehrere Polyethersiloxane und
c) einen oder mehrere von b) verschiedenen organischen Komplexliganden
d) einen oder mehrere von b) und c) verschiedene komplexbildende Komponenten, bevorzugt Polyether.

In einer weiterhin bevorzugten Ausführungsform der vorliegenden Erfindung enthalten DMC-Katalysatoren, die bevorzugt über das erfindungsgemäße Verfahren erhältlich sind,
a) ein oder mehrere Doppelmetallcyanid-Verbindungen ausgewählt aus Zinkhexacyanocobaltat(III), Zinkhexacyanoiridat (III), Zinkhexacyanoferrat(III) und Cobalt(II)hexacyanocobaltat(III),
b) ein oder mehrere Polyethersiloxane
c) einen oder mehrere von b) verschiedenen organischen Komplexliganden ausgewählt aus aliphatischen Alkoholen,
d) optional einen oder mehrere von b) und c) verschiedene komplexbildende Komponenten ausgewählt aus Polyether, Polyester, Polycarbonat, Glycidylether, Glycosid, Carbonsäureester mehrwertiger Alkohole, Polyalkylenglykolsorbitanester, Gallensäure, Salze der Gallensäure, Ester der Gallensäure, Amid der Gallensäure, Cyclodextrin, organisches Phophat, Phosphit, Phosphonat, Phosphonit, Phosphinat oder Phosphinit, eine ionische oberflächen- bzw. grenzflächenaktive Verbindung oder ein α,β-ungesättigter Carbonsäureester.

In einer ganz besonders bevorzugten Ausführungsform der vorliegenden Erfindung enthalten die DMC-Katalysatoren, die bevorzugt über das erfindungsgemäße Verfahren erhältlich sind,
a) eine Doppelmetallcyanid-Verbindung ausgewählt aus Zinkhexacyanocobaltat (III), Zinkhexacyanoferrat(III) und Cobalt(II)hexacyanocobaltat (III), und
b) ein oder mehrere Polyethersiloxane, bevorzugt der Formel (IV) bei denen bevorzugt die berechnete mittlere Molmasse des Polyethersiloxans von 500 bis 30.000 g/Mol , beträgt und das Polyethersiloxan ein Gewichtsverhältnis von Siloxan-Anteil zu Polyether- Anteil von 1 zu 1 bis 1 zu 10, bevorzugt 1 zu 1,2 bis 1 zu 8, besonders bevorzugt von 1 zu 1,5 bis 1 zu 5, und insbesondere bevorzugt von 1 zu 1,8 bis 1 zu 3, bezogen auf die berechnete mittlere Molmasse des Polyethersiloxans aufweist, besonders bevorzugt mit mit d = 2, d1 = 0, e = 0 bis 40, e1 = 1 bis 5, e2, f und g = 0, R¹ = Methyl, R² = (Formel V), (Formel VI) und/ oder (Formel VII) mit h = 0, j = 3 bis 20, k = 0 bis 20, bevorzugt 0, R' = Methyl oder Ethyl und R" = Wasserstoff;
c) ein oder mehrere aliphatische Alkohole, bevorzugt tert.-Butanol und
d) optional einen oder mehrere Polyether, bevorzugt Polyethylenglycole oder Polypropylenglycole.

"Über das erfindungsgemäße Verfahren erhältlich" bedeutet im Sinne dieser Erfindung, "erhältlich durch Umsetzung von wasserlöslichen Metallsalzen mit wasserlöslichen Metallcyanidsalzen zu Doppelmetallcyanid-Verbindungen der Komponente a), wobei die Umsetzung in Anwesenheit mindestens eines Polyethersiloxans der Komponente b) erfolgt." Bevorzugt sind die erfindungsgemäßen Katalysatoren "erhältlich indem wasserlösliche Metallsalze der Komponente a1), insbesondere der Formel (I), mit wasserlöslichen Metallcyanidsalzen der Komponente a2), insbesondere der Formel (II), in Anwesenheit mindestens eines Polyethersiloxans der Komponente b), insbesondere der Formel (IV), umgesetzt werden." Zudem werden alle Produkte umfasst, die durch das nachfolgend beschriebene Verfahren und bevorzugte Ausführungsformen davon erhältlich sind.

Das erfindungsgemäße Verfahren zur Herstellung von DMC-Katalysatoren zeichnet sich dadurch aus, dass während der Herstellung der Doppelmetallcyanid-Verbindung der Komponente a), welche ein Reaktionsprodukt wasserlöslicher Metallsalze der Komponente a1), und wasserlöslicher Metallcyanidsalze der Komponente a2) ist, mindestens ein Polyethersiloxan der Komponente b) anwesend also im Reaktionsgemisch enthalten sein muss. Die Bildung der Doppelmetallcyanid-Verbindung der Komponente a) erfolgt also in Gegenwart mindestens eines Polyethersiloxans der Komponente b).

Ein weiterer Gegenstand der vorliegenden Erfindung ist daher ein Verfahren zur Herstellung von DMC-Katalysatoren, bei dem wasserlösliche Metallsalze der Komponente a1), insbesondere der Formel (I), mit wasserlöslichen Metallcyanidsalzen der Komponente a2), insbesondere der Formel (II), in Anwesenheit mindestens eines Polyethersiloxans der Komponente b), insbesondere der Formel (IV), umgesetzt werden. Bevorzugt erfolgt die Umsetzung in Gegenwart von mindestens einem von b) verschiedenen organischen Komplexliganden der Komponente c). Für die bevorzugten Ausführungsformen der in diesem Verfahren eingesetzten Komponenten a), a1), a2), b) und gegebenenfalls auch c) und/oder d) gelten die zuvor beschriebenen Ausführungsformen der jeweiligen Komponenten. Überraschenderweise hat sich gezeigt, dass die Anwesenheit mindestens eines Polyethersiloxans der Komponente b) während der Umsetzung der Komponenten a1) und a2) ausschlaggebend ist für die hervorragenden Eigenschaften des Katalysators. In umfangreichen Studien konnte gezeigt werden, dass eine nachträgliche Zugabe von Polyethersiloxanen zu im Stand der Technik beschriebenen DMC-Katalysatoren, beziehungsweise zu auf dem Markt erhältlichen DMC-Katalysatoren nicht die Ergebnisse erzielen können, die erfindungsgemäße DMC-Katalysatoren erreichen.

Die Herstellung der DMC-Katalysatoren erfolgt üblicherweise in wässriger Lösung durch Umsetzung von Metallsalzen der Komponente a1), insbesondere der Formel (I) mit Metallcyanidsalzen der Komponente a2), insbesondere der Formel (II), in Gegenwart mindestens eines Polyethersiloxans der Komponente b) und gegebenenfalls in Gegenwart von organischen Komplexliganden der Komponente c) und/oder einer oder mehrerer weiterer komplexbildender Komponenten d).

Bevorzugt werden bei dem erfindungsgemäßen Verfahren zunächst die wässrigen Lösungen des Metallsalzes der Komponente a1), beispielsweise Zinkchlorid, bevorzugt eingesetzt im stöchiometrischen Überschuss (mindestens 50 Mol-% bezogen auf das Metallcyanidsalz) und des Metallcyanidsalzes der Komponente a2), beispielsweise Kaliumhexacyanocobaltat, in Gegenwart mindestens eines Polyethersiloxans der Komponente b) und gegebenenfalls in Gegenwart eines organischen Komplexliganden c), beispielsweise tert.-Butanol umgesetzt, wobei sich eine Dispersion bildet. Das Polyethersiloxans der Komponente b) sollte vorzugsweise entweder in der wässrigen Lösung des Metallsalzes der Komponente a1) und/oder des Metallcyanidsalzes der Komponente a2) vorhanden sein, kann aber prinzipiell auf jede andere Weise hinzugefügt werden, solange sichergestellt ist, dass das Polyethersiloxan der Komponente b) während der Bildung der Doppelmetallcyanid-Verbindungen der Komponente a), bevorzugt aus Komponente a1) und Komponente a2) anwesend ist. Der organische Komplexligand c) kann in der wässrigen Lösung des Metallsalzes der Komponente a1) und/oder des Metallcyanidsalzes der Komponente a2) vorhanden sein oder er wird der nach Ausfällung der Doppelmetallcyanid-Verbindung der Komponente a1) erhaltenen Dispersion unmittelbar zugegeben. Bevorzugt wird die gebildete Dispersion anschließend noch mit einer oder mehreren weiteren komplexbildenden Komponenten d) behandelt. Die weitere komplexbildende Komponente d) kann dabei bevorzugt in einer Mischung mit Wasser und organischem Komplexliganden c) eingesetzt werden.

Zur Abtrennung der Katalysatorpartikel aus der Dispersion können alle bekannten Verfahren zur Filtration oder Sedimentation eingesetzt werden. Die Abtrennung kann bevorzugt bei Temperaturen von 10°C bis 80°C durchgeführt werden. Die angelegten Druckdifferenzen können 0,001 bar bis 200 bar, bevorzugt 0,1 bar bis 100 bar, besonders bevorzugt 0,1 bar bis 25 bar betragen, wobei die eingesetzte Druckdifferenz von der eingesetzten Vorrichtung abhängig ist.

Anschließend kann der abgetrennte, aber gegebenenfalls noch durch wasserlösliche Nebenprodukte verunreinigte Katalysator gewaschen werden. Im erfindungsgemäßen Verfahren wird bevorzugt mit einer oder mehreren wässrigen Lösungen des Komplexliganden c) und ggf. einer oder mehreren weiteren komplexbildenden Komponenten d) eine Verdrängungswäsche durchgeführt. Besonders bevorzugt wird der feuchte Filterkuchen zunächst mit einer wässrigen Lösung des organischen Komplexliganden c) (z.B. tert.-Butanol) gewaschen. Auf diese Weise können wasserlösliche Nebenprodukte wie z.B. Kaliumchlorid aus dem Katalysator entfernt werden. Dabei beträgt die Menge des organischen Komplexliganden c) in der wässrigen Waschlösung bevorzugt 40 bis 80 Gew.-%, bezogen auf die Gesamtlösung. Bevorzugt kann es weiterhin sein, der wässrigen Waschlösung etwas an weiterer komplexbildender Komponente d) zuzufügen, besonders bevorzugt 0,5 bis 5 Gew.-%, bezogen auf die Gesamtlösung. Nach diesem ersten Waschschritt können sich weitere Waschschritte mit wässrigen oder nichtwässrigen Lösungen des organischen Komplexliganden c) und gegebenenfalls einer oder mehreren weiteren komplexbildenden Komponenten d) anschließen.

Die nach dem erfindungsgemäßen Verfahren hergestellten DMC-Katatysatoren können wegen ihrer außerordentlich hohen Aktivität häufig in sehr niedrigen Konzentrationen eingesetzt werden (25 ppm und weniger, bezogen auf die Menge des herzustellenden Polyetherpolyols).

Ein weiterer Gegenstand der vorliegenden Erfindung ist die Verwendung der erfindungsgemäßen DMC-Katalysatoren und der erfindungsgemäß hergestellten DMC-Katalysatoren als Katalysator bei der Herstellung von Polymeren, wie die Herstellung von Polyetherpolyolen, insbesondere bei der Alkoxylierung.

Der Gegenstand der vorliegenden Erfindung wird nachfolgend an Hand von Beispielen näher erläutert, ohne dass der Gegenstand der Erfindung auf diese beispielhaften Ausführungsformen beschränkt sein soll.

### Beispiele:

### Verwendete Chemikalien:

Zinkchlorid (≥ 98%) und Kaliumhexacyanocobaltat(III) wurden von Sigma-Aldrich bezogen. Tert-Butanol (≥ 99%) wurde von Carl Roth bezogen. Propylenoxid und Ethylenoxid wurde von GHC Gerling, Holz & CO Handels GmbH bezogen. Pluronic® 6100 wurde von BASF SE bezogen.

### GPC-Messungen:

GPC-Messungen zur Bestimmung der Polydispersität, gewichtsmittleren und zahlenmittleren Molmassen Mw und Mn wurden unter den folgenden Messbedingungen durchgeführt: Säulenkombination SDV 1000/10000 Å (Länge 65 cm), Temperatur 30 °C, THF als mobile Phase, Fließrate 1 ml/min, Probenkonzentration 10 g/l, RI-Detektor, Auswertung der Polyether erfolgte gegen Polypropylenglykol-Standard (76-6000 g/mol).

### Bestimmung der OH-Zahl:

Hydroxylzahlen wurden nach der Methode DGF C-V 17a (53) der deutschen Gesellschaft für Fettwissenschaft bestimmt. Dabei wurden die Proben mit Essigsäureanhydrid in Gegenwart von Pyridin acetyliert und der Verbrauch an Essisäureanhydrid durch Titration mit 0,5 N Kalilauge in Ethanol gegen Phenolphthalein bestimmt.

In den nachfolgenden Beispielen wurde als komplexbildende Komponente d) Polypropylenglycol (PPG)mit der Molmasse M_{OH} = 765 g/mol, die über die OH-Zahl ermittelt wurde, eingesetzt.

### Beispiel A: Herstellung der Doppelmetallcyanidkatalysatoren:

### Beispiel A1: (Vergleichsbeispiel) ohne Additiv

In einem Mehrhalskolben ausgestattet mit KPG-Blatt-Rührer, Rückflusskühler, Inertgaszuleitung und Temperaturfühler wurden 3,32 g Kaliumhexacyanocobaltat(III) gelöst in 40 mL dest. Wasser vorgelegt und unter Stickstoffatmosphere auf 50 °C erwärmt. Separat wurde eine Lösung aus 13,63 g Zinkchlorid, 20 mL tert-Butanol in 100 mL dest. Wasser hergestellt und mittels eines Tropftrichters in die vorgelegte Lösung innerhalb ca. einer Stunde zugetropft. Anschließend wurde eine weitere Lösung bestehend aus 3,5 g PPG, 1 mL dest. Wasser und 20 mL tert-Butanol zum Reaktionsgemisch zugetropft. Nach einer 10 minütigen Nachrührzeit wurde die entstandene Suspension filtriert. Der Filterkuchen wurde im Kolben in einer Lösung aus 40 mL dest. Wasser und 80 mL tert-Butanol aufgeschlämmt, erneut filtriert mit dem oben genannten tert-Butanol/Wasser-Gemisch gewaschen und anschließend bei 70 °C in einem Trockenschrank über Nacht getrocknet. Man enthielt als Produkt 4,93 g weißes Pulver, das über CaCl₂ im Exsikkator aufbewahrt wurde.

### Beispiel A2: DMC Herstellung unter Verwendung eines Polyethersiloxans mit 36 Gew-% Siloxan-Anteil und 64 Gew-% Polyether-Anteil

In einem Mehrhalskolben mit KPG-Blatt-Rührer, Rückflusskühler, Inertgaszuleitung und Temperaturfühler wurden 3,32 g Kaliumhexacyanocobaltat(III) gelöst in 40 mL dest. Wasser vorgelegt und unter Stickstoffatmosphere auf 50 °C erwärmt. Separat wurde eine Lösung aus 13,63 g Zinkchlorid, 20 mL tert-Butanol, 1 g eines Polyethersiloxans mit 36 Gew-% Siloxan-Anteil und 64 Gew-% Polyether-Anteil in 100 mL dest. Wasser hergestellt und mittels eines Tropftrichters in die vorgelegte Lösung innerhalb ca. einer Stunde zugetropft. Anschließend wurde eine weitere Lösung bestehend aus 3,5 g PPG, 1 mL dest. Wasser, 1 g eines Polyethersiloxans mit 36 Gew-% Siloxan-Anteil und 64 Gew-% Polyether-Anteil und 20 mL tert-Butanol zum Reaktionsgemisch zugetropft. Nach einer 10 minütigen Nachrührzeit wurde die entstandene Suspension filtriert. Der Filterkuchen wurde im Kolben in einer Lösung aus 40 mL dest. Wasser und 80 mL tert-Butanol aufgeschlämmt, erneut filtriert mit dem oben genannten tert-Butanol/Wasser-Gemisch gewaschen und anschließend bei 70 °C in einem Trockenschrank über Nacht getrocknet. Man enthielt als Produkt 5,18 g weißes Pulver, das über CaCl₂ im Exsikkator aufbewahrt wurde.

### Beispiel A3: DMC Herstellung unter Verwendung eines Polyethersiloxans mit 32 Gew-% Siloxan-Anteil und 68 Gew-% Polyether-Anteil

In einem Mehrhalskolben ausgestattet mit KPG-Blatt-Rührer, Rückflusskühler, Inertgaszuleitung und Temperaturfühler wurden 3,32 g Kaliumhexacyanocobaltat(III) gelöst in 40 mL dest. Wasser vorgelegt und unter Stickstoffatmosphere auf 50 °C erwärmt. Separat wurde eine Lösung aus 13,63 g Zinkchlorid, 20 mL tert-Butanol, 1 g eines Polyethersiloxans mit 32 Gew-% Siloxan-Anteil und 68 Gew-% Polyether-Anteil in 100 mL dest. Wasser hergestellt und mittels eines Tropftrichters in die vorgelegte Lösung innerhalb ca. einer Stunde zugetropft. Anschließend wurde eine weitere Lösung bestehend aus 3,5 g PPG, 1 mL dest. Wasser, 1 g eines Polyethersiloxans mit 32 Gew-% Siloxan-Anteil und 68 Gew-% Polyether-Anteil und 20 mL tert-Butanol zum Reaktionsgemisch zugetropft. Nach einer 10 minütigen Nachrührzeit wurde die entstandene Suspension filtriert. Der Filterkuchen wurde im Kolben in einer Lösung aus 40 mL dest. Wasser und 80 mL tert-Butanol aufgeschlämmt, erneut filtriert mit dem oben genannten tert-Butanol/WasserGemisch gewaschen und anschließend bei 70 °C in einem Trockenschrank über Nacht getrocknet. Man enthielt als Produkt 4,69 g weißes Pulver, das über CaCl₂ im Exsikkator aufbewahrt wurde.

### Beispiel A4: DMC Herstellung unter Verwendung eines Polyethersiloxans mit 32 Gew-% Siloxan-Anteil und 68 Gew-% Polyether-Anteil nur in PPG-Lösung (Vergleichsbeispiel)

In einem Mehrhalskolben ausgestattet mit KPG-Blatt-Rührer, Rückflusskühler, Inertgaszuleitung und Temperaturfühler wurden 3,32 g Kaliumhexacyanocobaltat(III) gelöst in 40 mL dest. Wasser vorgelegt und unter Stickstoffatmosphere auf 50 °C erwärmt. Separat wurde eine Lösung aus 13,63 g Zinkchlorid, 20 mL tert-Butanol in 100 mL dest. Wasser hergestellt und mittels eines Tropftrichters in die vorgelegte Lösung innerhalb ca. einer Stunde zugetropft. Anschließend wurde eine weitere Lösung bestehend aus 3,5 g PPG, 1 mL dest. Wasser, 1 g eines Polyethersiloxans mit 32 Gew-% Siloxan-Anteil und 68 Gew-% Polyether-Anteil und 20 mL tert-Butanol zum Reaktionsgemisch zugetropft. Nach einer 10 minütigen Nachrührzeit wurde die entstandene Suspension filtriert. Der Filterkuchen wurde im Kolben in einer Lösung aus 40 mL dest. Wasser und 80 mL tert-Butanol aufgeschlämmt, erneut filtriert mit dem oben genannten tert-Butanol/Wasser-Gemisch gewaschen und anschließend bei 70 °C in einem Trockenschrank über Nacht getrocknet. Man enthielt als Produkt 4,5 g weißes Pulver, das über CaCl₂ im Exsikkator aufbewahrt wurde.

### Beispiel A5: DMC Herstellung unter Verwendung von eines Polyethersiloxans mit 32 Gew-% Siloxan-Anteil und 68 Gew-% Polyether-Anteil nur in Waschlösung (Vergleichsbeispiel)

In einem Mehrhalskolben ausgestattet mit KPG-Blatt-Rührer, Rückflusskühler, Inertgaszuleitung und Temperaturfühler wurden 3,32 g Kaliumhexacyanocobaltat(III) gelöst in 40 mL dest. Wasser vorgelegt und unter Stickstoffatmosphere auf 50 °C erwärmt. Separat wurde eine Lösung aus 13,63 g Zinkchlorid, 20 mL tert-Butanol in 100 mL dest. Wasser hergestellt und mittels eines Tropftrichters in die vorgelegte Lösung innerhalb ca. einer Stunde zugetropft. Anschließend wurde eine weitere Lösung bestehend aus 3,5 g PPG, 1 mL dest. Wasser und 20 mL tert-Butanol zum Reaktionsgemisch zugetropft. Nach einer 10 minütigen Nachrührzeit wurde die entstandene Suspension filtriert. Der Filterkuchen wurde im Kolben in einer Lösung aus 40 mL dest. Wasser, 1 g eines Polyethersiloxans mit 32 Gew-% Siloxan-Anteil und 68 Gew-% Polyether-Anteil und 80 mL tert-Butanol aufgeschlämmt, erneut filtriert und anschließend bei 70 °C in einem Trockenschrank über Nacht getrocknet. Man enthielt als Produkt 5,07 g weißes Pulver, das über CaCl₂ im Exsikkator aufbewahrt wurde.

### Beispiel A6: DMC Herstellung unter Verwendung von Pluronic® 6100 (Vergleichsbeispiel)

In einem Mehrhalskolben ausgestattet mit KPG-Blatt-Rührer, Rückflusskühler, Inertgaszuleitung und Temperaturfühler wurden 3,32 g Kaliumhexacyanocobaltat(III) gelöst in 40 mL dest. Wasser vorgelegt und unter Stickstoffatmosphere auf 50 °C erwärmt. Separat wurde eine Lösung aus 13,63 g Zinkchlorid, 20 mL tert-Butanol, 1 g Pluronic® 6100 in 100 mL dest. Wasser hergestellt und mittels eines Tropftrichters in die vorgelegte Lösung innerhalb ca. einer Stunde zugetropft. Anschließend wurde eine weitere Lösung bestehend aus 3,5 g PPG, 1 mL dest. Wasser, 1 g Pluronic® 6100 und 20 mL tert-Butanol zum Reaktionsgemisch zugetropft. Nach einer 10 minütigen Nachrührzeit wurde die entstandene Suspension filtriert. Der Filterkuchen wurde im Kolben in einer Lösung aus 40 mL dest. Wasser und 80 mL tert-Butanol aufgeschlämmt, erneut filtriert mit dem oben genannten tert-Butanol/Wasser-Gemisch gewaschen und anschließend bei 70 °C in einem Trockenschrank über Nacht getrocknet. Man enthielt als Produkt 5,01 g weißes Pulver, das über CaCl₂ im Exsikkator aufbewahrt wurde.

### Beispiel B: Herstellung von Polyetherpolyolen:

In den nachfolgenden Beispielen wurde als Starterpolyether beispielhaft Poly(oxypropylen)-monobutylether mit der Molmasse M_{OH} = 384 g/mol, die über die OH-Zahl ermittelt wurde, eingesetzt. Prinzipiell können die Synthesen mit jedem Starter durchgeführt werden, der ein oder mehre Hydroxygruppen aufweist und der sich zum Einsatz in DMC-Katalysierten Reaktionen eignet.

Unter Induktionszeit wird die Zeit verstanden, in der die zur Aktivierung des Katalysators vorgelegte Startmenge des Propylenoxids (60 g) vollständig verbraucht wird. Der Verbrauch des Monomers wird mittels eines Druckmessgerätes verfolg. Der Verbrauch ist dann vollständig, wenn der Druck im Reaktor nach der Zugabe von PO auf den Anfangsdruck (der Druckwert bevor die Startmenge PO zugegeben wurde) zurückfällt. Die Gesamtreaktionszeit beinhaltet die Induktionsperiode und die Reaktionszeit die danach gebraucht wurde, um das restliche Monomer umzusetzen.

### Beispiel B1: Vergleichsbeispiel - Katalysator aus Beispiel A1

In einem 5 Liter Autoklaven wurden als Starter 255 g Poly(oxypropylen)-monobutylether (M= 384 g/mol) und 96 mg DMC-Katalysator A1 vorgelegt und unter Rühren auf 130 °C aufgeheizt. Der Reaktor wurde bis auf einen Innendruck von 30 mbar evakuiert, um evtl. vorhandene flüchtige Inhaltsstoffe destillativ zu entfernen. Zur Aktivierung des DMC-Katalysators wurde eine Portion von 60 g Propylenoxid zugeführt. Nach Anspringen der Reaktion und Innendruckabfall wurden unter Kühlen zunächst weitere 325 g Propylenoxid zudosiert. Es schloss sich eine 40-minütige Nachreaktion bei 130 °C mit nachfolgender Entgasung an. Dabei wurden flüchtige Anteile wie restliches Propylenoxid im Vakuum bei 130 °C abdestilliert. Das fast farblose Alkoxylierungsprodukt wurde auf unter 90 °C abgekühlt und aus dem Reaktor abgelassen.

Das Produkt hatte lt. GPC eine gewichstmittlere Molmasse von 1042 g/mol und eine Polydispersität Mw/Mn von 1,09. Die ermittelte OH-Zahl beträgt 61,0
Induktionsperiode: 72 min
Gesamtreaktionszeit: 139 min

### Beispiel B2: Katalysator aus Beispiel A2

Der Versuch wurde analog Beispiel 1 durchgeführt. Als Katalysator wurde DMC-Katalysator A2 eingesetzt.

Das Produkt hatte lt. GPC eine gewichtsmittlere Molmasse von 976 g/mol und eine Polydispersität Mw/Mn von 1,04. Die ermittelte OH-Zahl beträgt 55,0.
Induktionsperiode: 15 min
Gesamtreaktionszeit:89 min

### Beispiel B3: Katalysator aus Beispiel A3

Der Versuch wurde analog Beispiel 1 durchgeführt. Als Katalysator wurde DMC-Katalysator A3 eingesetzt.

Das Produkt hatte lt. GPC eine gewichtsmittlere Molmasse von 993 g/mol und eine Polydispersität Mw/Mn von 1,05. Die ermittelte OH-Zahl beträgt 58,9.
Induktionsperiode: 15 min
Gesamtreaktionszeit:53 min

### Beispiel B4: Katalysator aus Beispiel A4 (Vergleichsbeispiel)

Der Versuch wurde analog Beispiel 1 durchgeführt. Als Katalysator wurde DMC-Katalysator A4 eingesetzt.

Das Produkt hatte lt. GPC eine gewichtsmittlere Molmasse von 1002 g/mol und eine Polydispersität Mw/Mn von 1,11. Die ermittelte OH-Zahl beträgt 56,2.
Induktionsperiode: 33 min
Gesamtreaktionszeit:74 min

### Beispiel B5: Katalysator aus Beispiel A5 (Vergleichsbeispiel)

Der Versuch wurde analog Beispiel 1 durchgeführt. Als Katalysator wurde DMC-Katalysator A5 eingesetzt.

Das Produkt hatte lt. GPC eine gewichtsmittlere Molmasse von 1018 g/mol und eine Polydispersität Mw/Mn von 1,06. Die ermittelte OH-Zahl beträgt 54,6.
Induktionsperiode: 20 min
Gesamtreaktionszeit:74 min

Die Ergebnisse der Alkoxylierungsversuche zeigen, dass Reaktionen in denen erfindungsgemäße DMC-Katalysatoren A3 und A2 eingesetzt wurden, kürzere Induktionszeiten im Vergleich zum Katalysator ohne Zusatz an Polyethersiloxanen aufweisen. Außerdem zeigen Katalysatoren, bei der Synthese derer Polyethersiloxan nicht während der Fällung des Zinkhexacyanocobaltatcomplexes, sondern im späteren Schritt, zugegeben wurde, eine längere Induktionszeit und sind somit weniger katalytisch aktiv.

Zudem wurden Versuche durchgeführt, wobei zur Aktivierung des DMC-Katalysators eine Portion von 40 g Ethylenoxid zugeführt wurde.

### Beispiel B6: Katalysator aus Beispiel A1

In einem 5 Liter Autoklaven wurden als Starter 255 g Poly(oxypropylen)-monobutylether und 96 mg DMC-Katalysator A1 vorgelegt und unter Rühren auf 130 °C aufgeheizt. Der Reaktor wurde bis auf einen Innendruck von 30 mbar evakuiert, um evtl. vorhandene flüchtige Inhaltsstoffe destillativ zu entfernen. Zur Aktivierung des DMC-Katalysators wurde eine Portion von 40 g Ethylenoxid zugeführt. Nach Anspringen der Reaktion und Innendruckabfall wurden zunächst weitere 252 g Ethylenoxid zudosiert. Da keine Druckabnahme im Reaktor beobachtet wurde, wurde die Zugabe von Ethylenoxid abgebrochen und der Reaktor desodoriert.

### Beispiel B7: Katalysator aus Beispiel A2

In einem 5 Liter Autoklaven wurden als Starter 255 g Poly(oxypropylen)-monobutylether und 96 mg DMC-Katalysator A2 vorgelegt und unter Rühren auf 130 °C aufgeheizt. Der Reaktor wurde bis auf einen Innendruck von 30 mbar evakuiert, um evtl. vorhandene flüchtige Inhaltsstoffe destillativ zu entfernen. Zur Aktivierung des DMC-Katalysators wurde eine Portion von 40 g Ethylenoxid zugeführt. Nach Anspringen der Reaktion und Innendruckabfall wurden unter Kühlen zunächst weitere 252 g Ethylenoxid zudosiert. Es schloss sich eine 40-minütige Nachreaktion bei 130 °C mit nachfolgender Entgasung an. Dabei wurden flüchtige Anteile wie restliches Ethylenoxid im Vakuum bei 130 °C abdestilliert. Das Alkoxylierungsprodukt wurde auf unter 90 °C abgekühlt und aus dem Reaktor abgelassen.

Das Produkt hatte lt. GPC eine gewichstmittlere Molmasse von 783 g/mol und eine Polydispersität Mw/Mn von 1,1. Die ermittelte OH-Zahl beträgt 72,5.
Induktionsperiode: 27 min

### Beispiel B8: Katalysator aus Beispiel A3

In einem 5 Liter Autoklaven wurden als Starter 255 g Poly(oxypropylen)-monobutylether und 96 mg DMC-Katalysator A3 vorgelegt und unter Rühren auf 130 °C aufgeheizt. Der Reaktor wurde bis auf einen Innendruck von 30 mbar evakuiert, um evtl. vorhandene flüchtige Inhaltsstoffe destillativ zu entfernen. Zur Aktivierung des DMC-Katalysators wurde eine Portion von 40 g Ethylenoxid zugeführt. Nach Anspringen der Reaktion und Innendruckabfall wurden unter Kühlen zunächst weitere 252 g Ethylenoxid zudosiert. Es schloss sich eine 40-minütige Nachreaktion bei 130 °C mit nachfolgender Entgasung an. Dabei wurden flüchtige Anteile wie restliches Ethylenoxid im Vakuum bei 130 °C abdestilliert. Das Alkoxylierungsprodukt wurde auf unter 90 °C abgekühlt und aus dem Reaktor abgelassen.

Das Produkt hatte lt. GPC eine gewichstmittlere Molmasse von 810 g/mol und eine Polydispersität Mw/Mn von 1,09. Die ermittelte OH-Zahl beträgt 72,8.
Induktionsperiode: 24 min

### Beispiel B9: Katalysator aus Beispiel A6

In einem 5 Liter Autoklaven wurden als Starter 255 g Poly(oxypropylen)-monobutylether und 96 mg DMC-Katalysator A6 vorgelegt und unter Rühren auf 130 °C aufgeheizt. Der Reaktor wurde bis auf einen Innendruck von 30 mbar evakuiert, um evtl. vorhandene flüchtige Inhaltsstoffe destillativ zu entfernen. Zur Aktivierung des DMC-Katalysators wurde eine Portion von 40 g Ethylenoxid zugeführt. Nach Anspringen der Reaktion und Innendruckabfall wurden unter Kühlen weitere 252 g Ethylenoxid zudosiert, wobei im Verlauf der Polymerisation eine Abnahme der Reaktionsgeschwindigkeit zu verzeichnen war. Es schloss sich eine 40-minütige Nachreaktion bei 130 °C mit nachfolgender Entgasung an. Dabei wurden flüchtige Anteile wie restliches Ethylenoxid im Vakuum bei 130 °C abdestilliert. Das Alkoxylierungsprodukt wurde auf unter 90 °C abgekühlt und aus dem Reaktor abgelassen.

Das Produkt hatte lt. GPC eine gewichstmittlere Molmasse von 929 g/mol und eine Polydispersität Mw/Mn von 1,11. Die ermittelte OH-Zahl beträgt 69,0.
Induktionsperiode: 44 min

### Beispiel B10: Katalysator aus Beispiel A4

In einem 5 Liter Autoklaven wurden als Starter 255 g Poly(oxypropylen)-monobutylether und 96 mg DMC-Katalysator A4 vorgelegt und unter Rühren auf 130 °C aufgeheizt. Der Reaktor wurde bis auf einen Innendruck von 30 mbar evakuiert, um evtl. vorhandene flüchtige Inhaltsstoffe destillativ zu entfernen. Zur Aktivierung des DMC-Katalysators wurde eine Portion von 40 g Ethylenoxid zugeführt. Nach Anspringen der Reaktion und Innendruckabfall wurde unter Kühlen zunächst weiter Ethylenoxid zudosiert. Nach dem 252 g Ethylenoxid bereits zugegeben waren, fiel der Innendruck im Reaktor nicht vollständig auf den ursprünglichen Wert ab, sodass am Ende der Polymerisation eine Restmenge an EO abdestilliert werden musste.

### Beispiel B11: Katalysator aus Beispiel A5

In einem 5 Liter Autoklaven wurden als Starter 255 g Poly(oxypropylen)-monobutylether und 96 mg DMC-Katalysator A5 vorgelegt und unter Rühren auf 130 °C aufgeheizt. Der Reaktor wurde bis auf einen Innendruck von 30 mbar evakuiert, um evtl. vorhandene flüchtige Inhaltsstoffe destillativ zu entfernen. Zur Aktivierung des DMC-Katalysators wurde eine Portion von 40 g Ethylenoxid zugeführt. Da nach 130 min der Innendruck nicht auf den ursprünglichen Wert abfiel und auch nicht mehr abnahm, wurde die Restmenge an EO abdestilliert und die Polymerizationsreaktion abgebrochen.

Die Ergebnisse der Ethoxylierungsversuche zeigen, dass Reaktionen in denen DMC-Katalysatoren A3 und A2 eingesetzt wurden, kürzere Induktionszeiten im Vergleich zum Katalysator A6 aufwiesen, der unter Verwendung eines anderen Surfactant synthetisiert wurde. Auffällig ist außerdem, dass Katalysator A1, der ohne Zusatz an Polyethersiloxanen hergestellt wurde, keine katalytische Aktivität bei der Ethoxylierung zeigte. Genauso inaktiv waren Katalysatoren, bei der Synthese derer Polyethersiloxan nicht während der Fällung des Zinkhexacyanocobaltatcomplexes, sondern im späteren Schritt zugegeben wurde,

## Patentansprüche

1. DMC-Katalysator enthaltend
a) ein oder mehrere Doppelmetallcyanid-Verbindungen und
b) ein oder mehrere Polyethersiloxane,
wobei das Polyethersiloxan der Komponente b) ein Polyethersiloxan der Formel (IV) ist,
M_{d} M'_{d1} Dₑ D'ₑ₁ D"ₑ₂ T_{f} Q_{g} (Formel IV),
wobei
M = (R¹₃Si O_{1/2})
M' = (R²R¹₂ Si O_{1/2})
D = (R¹₂ Si O_{2/2})
D' = (R²R¹ Si O_{2/2})
D" = (R⁴R¹ Si O_{2/2})
T = (R³ Si O_{3/2})
Q = (Si O_{4/2})
d = 0 bis 20;
d1 = 0 bis 20;
e= 0 bis 300;
e1 = 0 bis 25;
e2 = 0 bis 10;
f = 0 bis 10;
g= 0 bis 10;
mit der Maßgabe, dass
die Summe aus d1 und e1 größer 0, bevorzugt größer gleich 1 ist; und
R¹ unabhängig voneinander Wasserstoff oder gleiche oder verschiedene lineare oder verzweigte Kohlenwasserstoffreste mit 1 bis 30 Kohlenstoffatomen oder aromatische Kohlenwasserstoffreste mit 6 bis 30 Kohlenstoffatomen;
R² unabhängig voneinander gleiche oder verschiedene Polyether, wobei die Polyether Seitenketten aufweisen können, die gegebenenfalls auch mit weiteren Heteroatomen substituiert sein können,
R³ unabhängig voneinander gleiche oder verschiedene Reste R¹ oder R²,
R⁴ unabhängig voneinander gleiche oder verschiedene organische Reste mit 4 bis 30 Kohlenstoffatomen, mit der Maßgabe, dass R⁴ verschieden von R² ist,
und wobei
der DMC-Katalysator erhältlich ist durch Umsetzung von wasserlöslichen Metallsalzen mit wasserlöslichen Metallcyanidsalzen zu Doppelmetallcyanid-Verbindungen der Komponente a), wobei die Umsetzung in Anwesenheit mindestens eines Polyethersiloxans der Komponente b) erfolgt.

2. DMC-Katalysator nach Anspruch 1, **dadurch gekennzeichnet, dass** das Polyethersiloxan der Komponente b) ein Gewichtsverhältnis von Siloxan- Anteil zu Polyether-Anteil von 1 zu 1 bis 1 zu 10, bevorzugt 1 zu 1,2 bis 1 zu 8, besonders bevorzugt von 1 zu 1,5 bis 1 zu 5, und insbesondere bevorzugt von 1 zu 1,8 bis 1 zu 3, bezogen auf die berechnete mittlere Molmasse des Polyethersiloxans aufweist.

3. DMC-Katalysator nach Anspruch 1 oder 2, **dadurch gekennzeichnet, dass** R² in Formel (IV) unabhängig voneinander gleiche oder verschiedene Polyether der Formel V oder Formel VI oder Formel VII
-(O)ₕ-CH₂-CH₂-CH₂-O-(CH₂-CH₂O-)ⱼ-(CH₂-CH(R')O-)ₖ-(CH(R')-CH₂O-)_{k'}-R" (Formel V)
und
-(O)ₕ-CH₂-CH₂-O-(CH₂-CH₂O-)ⱼ-(CH₂-CH(R')O-)ₖ-(CH(R')-CH₂O-)_{k'}-R" (Formel VI)
-(O)ₕ-(CH₂-CH_{2O}-)ⱼ-(CH₂-CH(R')O-)ₖ-(CH(R')-CH₂O-)_{k'}-R" (Formel VII)
mit
h 0 oder 1
j 0 bis 100,
k + k' = 0 bis 100,
mit der Maßgabe, dass die Summe aus j, k und k' mindestens 3 ergibt,
R' unabhängig voneinander eine gegebenenfalls substituierte Alkyl- oder Arylgruppe mit 1 bis 12 C-Atomen und
R" unabhängig voneinander ein Wasserstoffrest oder eine Alkylgruppe mit 1 bis 4 C-Atomen,
bedeutet.

4. DMC-Katalysator einem der Ansprüche 1 bis 3, **dadurch gekennzeichnet, dass** die Polyethersiloxane der Komponente b) Polyethersiloxane der Formel (IV) mit d = 2, d1 = 0, e = 0 bis 40, e1 = 1 bis 5, e2, f und g = 0, R1 = Methyl, R2 = (Formel V), (Formel VI) und/ oder (Formel VII) mit h = 0, j = 3 bis 20, k = 0 bis 20, bevorzugt 0, R' = Methyl oder Ethyl und R" = Wasserstoff, sind.

5. DMC-Katalysator nach einem der Ansprüche 1 bis 4, **dadurch gekennzeichnet, dass** die Doppelmetallcyanid-Verbindungen der Komponente a) Verbindungen der allgemeinen Formel (III)
Mₓ[M'_{x'}(CN)_{y}]_{z} (III)
wobei
M ausgewählt wird aus den Metallen Zn(II), Fe(II), Co(II) oder Ni(II), M' ausgewählt wird aus den Metallen Co(III), Fe(III), Cr(III) oder Ir(III) mit x = 3, x' = 1, y = 6 und z = 2
sind.

6. DMC-Katalysator nach einem der Ansprüche 1 bis 5 enthaltend weiterhin als Komponente c) einen oder mehrere von b) verschiedenen organischen Komplexliganden ausgewählt aus der Gruppe der Alkohole, Aldehyde, Ketone, Ether, Ester, Amide, Harnstoffe, Nitrile, Sulfide und deren Mischungen.

7. DMC-Katalysatoren nach einem der Ansprüche 1 bis 6 enthaltend
a) ein oder mehrere Doppelmetallcyanid-Verbindungen ausgewählt aus Zinkhexacyanocobaltat(III), Zinkhexacyanoiridat (III), Zinkhexacyanoferrat(III) und Cobalt(II)hexacyanocobaltat(III),
b) ein oder mehrere Polyethersiloxane
c) einen oder mehrere von b) verschiedenen organischen Komplexliganden ausgewählt aus aliphatischen Alkoholen,
d) optional einen oder mehrere von b) und c) verschiedene komplexbildende Komponenten ausgewählt aus Polyether, Polyester, Polycarbonat, Glycidylether, Glycosid, Carbonsäureester mehrwertiger Alkohole, Polyalkylenglykolsorbitanester, Gallensäure, Salze der Gallensäure, Ester der Gallensäure, Amid der Gallensäure, Cyclodextrin, organisches Phophat, Phosphit, Phosphonat, Phosphonit, Phosphinat oder Phosphinit, eine ionische oberflächen- bzw. grenzflächenaktive Verbindung oder ein α,β-ungesättigter Carbonsäureester.

8. Verfahren zur Herstellung von DMC-Katalysatoren wobei wasserlösliche Metallsalze mit wasserlöslichen Metallcyanidsalzen zu Doppelmetallcyanid-Verbindungen der Komponente a) umgesetzt werden, **dadurch gekennzeichnet, dass** die Umsetzung in Anwesenheit mindestens eines Polyethersiloxans der Komponente b) erfolgt.

9. Verfahren nach Anspruch 8, **dadurch gekennzeichnet, dass** das Polyethersiloxan der Komponente b) ein Gewichtsverhältnis von Siloxan- Anteil zu Polyether-Anteil von 1 zu 1 bis 1 zu 10, bevorzugt 1 zu 1,2 bis 1 zu 8, besonders bevorzugt von 1 zu 1,5 bis 1 zu 5, und insbesondere bevorzugt von 1 zu 1,8 bis 1 zu 3, bezogen auf die berechnete mittlere Molmasse des Polyethersiloxans, aufweist.

10. Verfahren nach einem der Ansprüche 8 oder 9, **dadurch gekennzeichnet, dass** wasserlösliche Metallsalze der Komponente a1) der Formel (I)
M(X)ₙ Formel (I),
mit
M ausgewählt aus Zn (II), Fe (II), Co (II) und Ni (II)
X gleiche oder verschiedene Anionen ausgewählt aus der Gruppe der Halogenide, Hydroxide, Sulfate, Carbonate, Cyanate, Thiocyanate, Isocyanate, Isothiocyanate, Carboxylate, Oxalate oder Nitrate und
n = 2;
mit wasserlöslichen Metallcyanidsalzen der Komponente a2) der Formel (II)
(Y)ₐM'(CN)_{b}(A)_{c} (II)
mit
M' eines oder mehrere Metalle ausgewählt aus Co(II), Co(III), Fe(II), Fe(III), Cr(III), Ir(III) und Ni(II),
Y gleiche oder verschiedene, vorzugsweise gleiche Alkalimetallkationen oder Erdalkalimetallkationen,
A gleiche oder verschiedene Anionen, ausgewählt aus der Gruppe der Halogenide, Hydroxide, Sulfate, Carbonate, Cyanate, Thiocyanate, Isocyanate, Isothiocyanate, Carboxylate, Oxalate oder Nitrate.
a ist 1, 2, 3 oder 4; b ist 4, 5 oder 6; c ist 0, 1 oder 2, wobei die Werte für a, b und c so gewählt sind, dass die Elektroneutralität des Metallcyanidsalzes gegeben ist;
in Anwesenheit mindestens eines Polyethersiloxans der Komponente b), der Formel (IV)
M_{d} M'_{d1} Dₑ D'ₑ₁ D"ₑ₂ T_{f} Q_{g} (Formel IV)
wobei
M = (R¹₃ Si O_{1/2})
M' = (R²R¹₂Si O_{1/2})
D = (R¹₂ Si O_{2/2})
D' = (R²R¹ Si O_{2/2})
D" = (R⁴R¹ Si O_{2/2})
T = (R³ Si O_{3/2})
Q = (Si O_{4/2})
d = 0 bis 20; bevorzugt 1 bis 10, besonders bevorzugt 1 bis 5 und insbesondere bevorzugt 2;
d1 = 0 bis 20; bevorzugt 1 bis 10, besonders bevorzugt 0 bis 2; insbesondere bevorzugt 0;
wobei die Summe aus d und d1 bevorzugt 2 ergibt;
e = 0 bis 300; bevorzugt 1 bis 100, besonders bevorzugt 2 bis 40, insbesondere bevorzugt 0 bis 20;
e1 = 0 bis 25; bevorzugt 0,1 bis 15, besonders bevorzugt 1 bis 10, insbesondere bevorzugt 1 bis 5;
e2 = 0 bis 10; bevorzugt 1 bis 5, insbesondere bevorzugt 0;
f = 0 bis 10; bevorzugt 1 bis 5, insbesondere bevorzugt 0;
g = 0 bis 10; bevorzugt 1 bis 5, insbesondere bevorzugt 0;
mit der Maßgabe, dass
die Summe aus d1 und e1 größer 0, bevorzugt größer gleich 1 ist; und
R¹ unabhängig voneinander gleiche oder verschiedene lineare oder verzweigte Kohlenwasserstoffreste mit 1 bis 30 Kohlenstoffatomen oder aromatische Kohlenwasserstoffreste mit 6 bis 30 Kohlenstoffatomen;
R² unabhängig voneinander gleiche oder verschiedene Polyether, wobei die Polyether Seitenketten aufweisen können, die gegebenenfalls auch mit weiteren Heteroatomen substituiert sein können,
R³ unabhängig voneinander gleiche oder verschiedene Reste R¹ oder R²;
R⁴ unabhängig voneinander gleiche oder verschiedene organische Reste mit 4 bis 30 Kohlenstoffatomen, mit der Maßgabe, dass R⁴ verschieden von R² ist,
und optional in Anwesenheit mindestens eines von b) verschiedenen organischen Komplexliganden der Komponente c) ausgewählt aus der Gruppe der Alkohole, Aldehyde, Ketone, Ether, Ester, Amide, Harnstoffe, Nitrile, Sulfide und deren Mischungen; umgesetzt werden.

11. Verfahren nach einem der Ansprüche 8 bis 10, **dadurch gekennzeichnet, dass** R² in Formel (IV) unabhängig voneinander gleiche oder verschiedene Polyether der Formel V oder Formel VI oder Formel VII
-(O)ₕ-CH₂-CH₂-CH₂-O-(CH₂-CH_{2O}-)ⱼ-(CH₂-CH(R')O-)ₖ-(CH(R')-CH₂O-)_{k'}-R" (Formel V)
und
-(O)ₕ-CH₂-CH₂-O-(CH₂-CH₂O-)ⱼ-(CH₂-CH(R')O-)ₖ-(CH(R')-CH₂O-)_{k'}-R" (Formel VI)
-(O)ₕ-(CH₂-CH₂O-)ⱼ-(CH₂-CH(R')O-)ₖ-(CH(R')-CH₂O-)_{k'}-R" (Formel VII)
h 0 oder 1,
j 0 bis 100,
k + k' = 0 bis 100,
mit
mit der Maßgabe, dass die Summe aus j und k mindestens 3 ergibt,
R' unabhängig voneinander eine gegebenenfalls substituierte Alkyl- oder Arylgruppe mit 1 bis 12 C-Atomen und
R" unabhängig voneinander ein Wasserstoffrest oder eine Alkylgruppe mit 1 bis 4 C-Atomen,
bedeutet.

12. Verfahren nach Anspruch 10, **dadurch gekennzeichnet, dass** wasserlösliche Metallsalze ausgewählt aus Zinkchlorid, Zinkbromid, Zinkacetat, Zinkacetylacetonat, Zinkbenzoat, Zinknitrat, Eisen(II)sulfat, Eisen(II)bromid, Eisen(II)chlorid, Cobalt(II)chlorid, Cobalt(II)thiocyanat, Nickel(II)chlorid, Nickel(II)nitrat und Mischungen daraus mit wasserlöslichen Metallcyanidsalzen ausgewählt aus Kaliumhexacyanocobaltat(III), Kaliumhexacyanoferrat(II), Kaliumhexacyano-ferrat(III), Calciumhexacyanocobaltat(III), Lithiumhexacyanocobaltat(III).und Mischungen daraus zu Doppelmetallcyanid-Verbindungen der Komponente a) umgesetzt werden, wobei die Umsetzung in Anwesenheit mindestens eines Polyethersiloxans der Komponente b) erfolgt und die Polyethersiloxane der Komponente b) optional Polyethersiloxane der Formel (IV) mit d = 2, d1 = 0, e = 0 bis 40, e1 = 1 bis 5, e2, f und g = 0, R1 = Methyl, R2 = (Formel V), (Formel VI) und/ oder (Formel VII) mit h = 0, j = 3 bis 20, k = 0 bis 20, bevorzugt 0, R' = Methyl oder Ethyl und R" = Wasserstoff sind.

13. Verwendung der DMC-Katalysatoren nach einem der Ansprüche 1 bis 7 oder der durch ein Verfahren gemäß einem der Ansprüche 8 bis 12 erhaltenen Katalysatoren bei der Herstellung von Polymeren.

## Claims

1. DMC catalyst comprising
a) one or more double metal cyanide compounds and
b) one or more polyether siloxanes,
wherein the polyether siloxane of component b) is a polyether siloxane of the formula (IV)
M_{d} M'_{d1} Dₑ D'ₑ₁ D''ₑ₂ T_{f} Q_{g} (formula IV)
where
M = (R¹₃ Si O_{1/2})
M' = (R²R¹₂ Si O_{1/2})
D = (R¹₂ Si O_{2/2})
D' = (R²R¹ Si O_{2/2})
D" = (R⁴R¹ Si O_{2/2})
T = (R³ Si O_{3/2})
Q = (Si O_{4/2})
d = 0 to 20;
d1 = 0 to 20;
e = 0 to 300;
e1 = 0 to 25;
e2 = 0 to 10;
f = 0 to 10;
g = 0 to 10;
with the proviso that
the sum total of d1 and e1 is greater than 0, preferably greater than or equal to 1;
and
R¹ is independently hydrogen or identical or different linear or branched hydrocarbyl radicals having 1 to 30 carbon atoms or aromatic hydrocarbyl radicals having 6 to 30 carbon atoms;
R² is independently identical or different polyethers, where the polyethers may have side chains which may optionally also be substituted by further heteroatoms,
R³ is independently identical or different R¹ or R² radicals,
R⁴ is independently identical or different organic radicals having 4 to 30 carbon atoms, with the proviso that R⁴ is different from R²,
and wherein
the DMC catalyst is obtainable by reacting water-soluble metal salts with water-soluble metal cyanide salts to give double metal cyanide compounds of component a), wherein the reaction is effected in the presence of at least one polyether siloxane of component b).

2. DMC catalyst according to Claim 1, **characterized in that** the polyether siloxane of component b) has a weight ratio of siloxane component to polyether component of 1:1 to 1:10, preferably 1:1.2 to 1:8, more preferably from 1:1.5 to 1:5, and especially preferably from 1:1.8 to 1:3, based on the calculated mean molar mass of the polyether siloxane.

3. DMC catalyst according to Claim 1 or 2, **characterized in that** R² in formula (IV) is independently identical or different polyethers of the formula V or formula VI or formula VII
-(O)ₕ-CH₂-CH₂-CH₂-O-(CH₂-CH₂O-)ⱼ-(CH₂-CH(R')O-)ₖ-(CH(R')-CH₂O-)_{k'}-R" (formula V)
and
- (O)ₕ-CH₂-CH₂-O-(CH₂-CH₂O-)ⱼ-(CH₂-CH(R')O-)ₖ-(CH(R')-CH₂O-)_{k'}-R" (formula VI)
- (O)ₕ-(CH₂-CH₂O-)ⱼ-(CH₂-CH(R')O-)ₖ-(CH(R')-CH₂O-)_{k'}-R" (formula VII)
where
h is 0 or 1,
j is 0 to 100,
k + k' = 0 to 100,
with the proviso that the sum total of j, k and k' is at least 3,
R' is independently an optionally substituted alkyl or aryl group having 1 to 12 carbon atoms and
R" is independently a hydrogen radical or an alkyl group having 1 to 4 carbon atoms.

4. DMC catalyst any of Claims 1 to 3, **characterized in that** the polyether siloxanes of component b) are polyether siloxanes of the formula (IV) with d = 2, d1 = 0, e = 0 to 40, e1 = 1 to 5, e2, f and g = 0, R1 = methyl, R2 = (formula V), (formula VI) and/or (formula VII) with h = 0, j = 3 to 20, k = 0 to 20, preferably 0, R' = methyl or ethyl and R" = hydrogen.

5. DMC catalyst according to any of Claims 1 to 4, **characterized in that** the double metal cyanide compounds of component a) are compounds of the general formula (III)
Mₓ[M'_{x'}(CN)_{y}]_{z} (III)
where
M is selected from the metals Zn(II), Fe(II), Co(ll) and Ni(ll),
M' is selected from the metals Co(III), Fe(III), Cr(lll) or Ir(III),
with x = 3, x' = 1, y = 6 and z = 2.

6. DMC catalyst according to any of Claims 1 to 5, further comprising, as component c), one or more organic complex ligands other than b), selected from the group of the alcohols, aldehydes, ketones, ethers, esters, amides, ureas, nitriles, sulphides and mixtures thereof.

7. DMC catalysts according to any of Claims 1 to 6, comprising
a) one or more double metal cyanide compounds selected from zinc hexacyanocobaltate(III), zinc hexacyanoiridate(III), zinc hexacyanoferrate(III) and cobalt(II) hexacyanocobaltate(III),
b) one or more polyether siloxanes,
c) one or more organic complex ligands other than b), selected from aliphatic alcohols,
d) optionally one or more complex-forming components other than b) and c), selected from polyethers, polyesters, polycarbonate, glycidyl ethers, glycoside, carboxylic esters of polyhydric alcohols, polyalkylene glycol sorbitan esters, bile acid, salts of bile acid, esters of bile acid, amide of bile acid, cyclodextrins, organic phosphate, phosphite, phosphonate, phosphonite, phosphinate or phosphinite, an ionic surface- or interface-active compound and an α,β-unsaturated carboxylic ester.

8. Process for preparing DMC catalysts wherein water-soluble metal salts are reacted with water-soluble metal cyanide salts to give double metal cyanide compounds of component a), **characterized in that** the reaction is effected in the presence of at least one polyether siloxane of component b).

9. Process according to Claim 8, **characterized in that** the polyether siloxane of component b) has a weight ratio of siloxane component to polyether component of 1:1 to 1:10, preferably 1:1.2 to 1:8, more preferably from 1:1.5 to 1:5, and especially preferably from 1:1.8 to 1:3, based on the calculated mean molar mass of the polyether siloxane.

10. Process according to either of Claims 8 and 9, **characterized in that** water-soluble metal salts of component a1) of the formula (I)
M(X)ₙ formula (I)
where
M is selected from Zn(II), Fe(II), Co(II) and Ni(II),
X is identical or different anions selected from the group of the halides, hydroxides, sulphates, carbonates, cyanates, thiocyanates, isocyanates, isothiocyanates, carboxylates, oxalates and nitrates and
n = 2;
are reacted with water-soluble metal cyanide salts of component a2) of the formula (II)
(Y)ₐM'(CN)_{b}(A)_{c} (II)
where
M' is one or more metals selected from Co(II), Co(III), Fe(II), Fe(III), Cr(lll), Ir(lll) and Ni(II),
Y is identical or different, preferably identical, alkali metal cations or alkaline earth metal cations,
A is identical or different anions selected from the group of the halides, hydroxides, sulphates, carbonates, cyanates, thiocyanates, isocyanates, isothiocyanates, carboxylates, oxalates and nitrates,
a is 1, 2, 3 or 4; b is 4, 5 or 6; c is 0, 1 or 2, where the values of a, b and c are chosen so as to give electronic neutrality of the metal cyanide salt;
in the presence of at least one polyether siloxane of component b), of the formula (IV)
M_{d}M'_{d1} DₑD'ₑ₁D"ₑ₂T_{f}Q_{g} (formula IV)
where
M = (R¹₃ Si O_{1/2})
M' = (R²R¹₂ Si O_{1/2})
D = (R¹₂ Si O_{2/2})
D' = (R²R¹ Si O_{2/2})
D" = (R⁴R¹ Si O_{2/2})
T = (R³ Si O_{3/2})
Q = (Si O_{4/2})
d = 0 to 20; preferably 1 to 10, more preferably 1 to 5 and especially preferably 2;
d1 = 0 to 20; preferably 1 to 10, more preferably 0 to 2; especially preferably 0;
where the sum total of d and d1 is preferably 2;
e = 0 to 300; preferably 1 to 100, more preferably 2 to 40, especially preferably 0 to 20;
e1 = 0 to 25; preferably 0.1 to 15, more preferably 1 to 10, especially preferably 1 to 5;
e2 = 0 to 10; preferably 1 to 5, especially preferably 0;
f = 0 to 10; preferably 1 to 5, especially preferably 0;
g = 0 to 10; preferably 1 to 5, especially preferably 0;
with the proviso that
the sum total of d1 and e1 is greater than 0, preferably greater than or equal to 1;
and
R¹ is independently identical or different linear or branched hydrocarbyl radicals having 1 to 30 carbon atoms or aromatic hydrocarbyl radicals having 6 to 30 carbon atoms;
R² is independently identical or different polyethers, where the polyethers may have side chains which may optionally also be substituted by further heteroatoms,
R³ is independently identical or different R¹ or R² radicals,
R⁴ is independently identical or different organic radicals having 4 to 30 carbon atoms, with the proviso that R⁴ is different from R²,
and optionally in the presence of at least one organic complex ligand of component c) other than b), selected from the group of the alcohols, aldehydes, ketones, ethers, esters, amides, ureas, nitriles, sulphides and mixtures thereof.

11. Process according to any of Claims 8 to 10, **characterized in that** R² in formula (IV) is independently identical or different polyethers of the formula V or formula VI or formula VII
-(O)ₕ-CH₂-CH₂-CH₂-O-(CH₂-CH₂O-)ⱼ-(CH₂-CH(R')O-)ₖ-(CH(R')-CH₂O-)_{k'}-R" (formula V)
and
- (O)ₕ-CH₂-CH₂-O-(CH₂-CH₂O-)ⱼ-(CH₂-CH(R')O-)ₖ-(CH(R')-CH₂O-)_{k'}-R" (formula VI)
- (O)ₕ-(CH₂-CH₂O-)ⱼ-(CH₂-CH(R')O-)ₖ-(CH(R')-CH₂O-)_{k'}-R" (formula VII)
h is 0 or 1,
j is 0 to 100,
k + k' = 0 to 100,
where
with the proviso that the sum total of j and k is at least 3,
R' is independently an optionally substituted alkyl or aryl group having 1 to 12 carbon atoms and
R" is independently a hydrogen radical or an alkyl group having 1 to 4 carbon atoms.

12. Process according to Claim 10, **characterized in that** water-soluble metal salts selected from zinc chloride, zinc bromide, zinc acetate, zinc acetylacetonate, zinc benzoate, zinc nitrate, iron(ll) sulfate, iron(ll) bromide, iron(ll) chloride, cobalt(ll) chloride, cobalt(ll) thiocyanate, nickel (11)chloride, nickel (11)nitrate and mixtures thereof with water-soluble metal cyanide salts selected from potassium hexacyanocobaltate(III), potassium hexacyanoferrate(II), potassium hexacyanoferrate(III), calcium hexacyanocobaltate(lll), lithium hexacyanocobaltate(III) and mixtures thereof are converted to double metal cyanide compounds of component a), the reaction being effected in the presence of at least one polyether siloxane of component b) and the polyether siloxanes of component b) optionally being polyether siloxanes of the formula (IV) with d = 2, d1 = 0, e = 0 to 40, e1 = 1 to 5, e2, f and g = 0, R1 = methyl, R2 = (formula V), (formula VI) and/or (formula VII) with h = 0, j = 3 to 20, k = 0 to 20, preferably 0, R' = methyl or ethyl and R" = hydrogen.

13. Use of the DMC catalysts according to any of Claims 1 to 7 or of the catalysts obtained by a process according to any of Claims 8 to 12 in the preparation of polymers.

## Revendications

1. Catalyseur DMC, contenant :
a) un ou plusieurs composés de cyanure de deux métaux et
b) un ou plusieurs polyéther-siloxanes,
le polyéther-siloxane du composant b) étant un polyéther-siloxane de formule (IV) :
M_{d}M'_{d1}DₑD'ₑ₁D"ₑ₂T_{f}Q_{g} (Formule IV)
dans laquelle
M = (R¹₃SiO_{1/2})
M' = (R²R¹₂SiO_{1/2})
D = (R¹₂SiO_{2/2})
D' = (R²R¹SiO_{2/2})
D" = (R⁴R¹SiO_{2/2})
T = (R³SiO_{3/2})
Q = (SiO_{4/2})
d = 0 à 20 ;
d1 = 0 à 20 ;
e = 0 à 300 ;
e1 = 0 à 25 ;
e2 = 0 à 10 ;
f = 0 à 10 ;
g = 0 à 10 ;
à condition que
la somme de d1 et e1 soit supérieure à 0, de préférence supérieure ou égale à 1 ;
et
les R¹ sont indépendamment les uns des autres l'hydrogène ou des radicaux hydrocarbonés linéaires ou ramifiés identiques ou différents de 1 à 30 atomes de carbone ou des radicaux hydrocarbonés aromatiques de 6 à 30 atomes de carbone ;
les R² sont indépendamment les uns des autres des polyéthers identiques ou différents, les polyéthers pouvant comprendre des chaînes latérales, qui peuvent éventuellement également être substituées avec des hétéroatomes supplémentaires,
les R³ sont indépendamment les uns des autres des radicaux R¹ ou R² identiques ou différents,
les R⁴ sont indépendamment les uns des autres des radicaux organiques identiques ou différents de 4 à 30 atomes de carbone, à condition que R⁴ soit différent de R²,
et
le catalyseur DMC pouvant être obtenu par mise en réaction de sels métalliques solubles dans l'eau avec des sels de cyanure métalliques solubles dans l'eau pour former les composés de cyanure de deux métaux du composant a), la réaction ayant lieu en présence d'au moins un polyéther-siloxane du composant b).

2. Catalyseur DMC selon la revendication 1, **caractérisé en ce que** le polyéther-siloxane du composant b) présente un rapport en poids entre la proportion de siloxane et la proportion de polyéther de 1 sur 1 à 1 sur 10, de préférence de 1 sur 1,2 à 1 sur 8, de manière particulièrement préférée de 1 sur 1,5 à 1 sur 5, et de manière notamment préférée de 1 sur 1,8 à 1 sur 3, par rapport à la masse molaire moyenne calculée du polyéther-siloxane.

3. Catalyseur DMC selon la revendication 1 ou 2, **caractérisé en ce que** les R² dans la formule (IV) sont indépendamment les uns des autres des polyéthers identiques ou différents de formule V ou de formule VI ou de formule VII
- (O)ₕ-CH₂-CH₂-CH₂-O-(CH₂-CH₂O-)ⱼ-(CH₂-CH(R')O-)ₖ-(CH(R')-CH₂O-)_{k'}-R" (Formule V)
et
-(O)ₕ-CH₂-CH₂-O-(CH₂-CH₂O-)ⱼ-(CH₂-CH(R')O-)ₖ-(CH(R')-CH₂O-)_{k'}-R" (Formule VI)
-(Oₕ-(CH₂-CH₂O-)ⱼ-(CH₂-CH(R')O-)ₖ-(CH(R')-CH₂O-)_{k'}-R" (Formule VII)
avec
h = 0 ou 1,
j = 0 à 100,
k + k' = 0 à 100,
à condition que la somme de j, k et k' soit d'au moins 3,
les R' signifient indépendamment les uns des autres un groupe alkyle ou aryle éventuellement substitué de 1 à 12 atomes C, et
les R" signifient indépendamment les uns des autres un radical hydrogène ou un groupe alkyle de 1 à 4 atomes C.

4. Catalyseur DMC l'une quelconque des revendications 1 à 3, **caractérisé en ce que** les polyéther-siloxanes du composant b) sont des polyéther-siloxanes de formule (IV) avec d = 2, d1 = 0, e = 0 à 40, e1 = 1 à 5, e2, f et g = 0, R1 = méthyle, R2 = (Formule V), (Formule VI) et/ou (Formule VII) avec h = 0, j = 3 à 20, k = 0 à 20, de préférence 0, R' = méthyle ou éthyle et R" = hydrogène.

5. Catalyseur DMC selon l'une quelconque des revendications 1 à 4, **caractérisé en ce que** les composés de cyanure de deux métaux du composant a) sont des composés de formule générale (III)
Mₓ[M'_{x'}(CN)_{y}]_{z} (III)
dans laquelle
M est choisi parmi les métaux Zn(II), Fe(II), Co(II) ou Ni (II),
M' est choisi parmi les métaux Co(III), Fe(III), Cr(III) ou Ir(III),
avec x = 3, x' = 1, y = 6 et z = 2.

6. Catalyseur DMC selon l'une quelconque des revendications 1 à 5, contenant en outre en tant que composant c) un ou plusieurs ligands complexes organiques différents de b) choisis dans le groupe constitué par les alcools, les aldéhydes, les cétones, les éthers, les esters, les amides, les urées, les nitriles, les sulfures et leurs mélanges.

7. Catalyseurs DMC selon l'une quelconque des revendications 1 à 6, contenant :
a) un ou plusieurs composés de cyanure de deux métaux choisis parmi l'hexacyanocobaltate de zinc (III), l'hexacyanoiridate de zinc (III), l'hexacyanoferrate de zinc (III) et l'hexacyanocobaltate (III) de cobalt (II),
b) un ou plusieurs polyéther-siloxanes,
c) un ou plusieurs ligands complexes organiques différents de b) choisis parmi les alcools aliphatiques,
d) éventuellement un ou plusieurs composants complexants différents de b) et c) choisis parmi un polyéther, un polyester, un polycarbonate, un éther glycidylique, un glycoside, un ester d'acide carboxylique d'alcools polyvalents, un ester de sorbitane de polyalkylène glycol, l'acide biliaire, un sel de l'acide biliaire, un ester de l'acide biliaire, un amide de l'acide biliaire, la cyclodextrine, un phosphate, phosphite, phosphonate, phosphonite, phosphinate ou phosphinite organique, un composé tensioactif ionique ou un ester d'acide carboxylique α,β-insaturé.

8. Procédé de fabrication de catalyseurs DMC, selon lequel des sels métalliques solubles dans l'eau sont mis en réaction avec des sels de cyanure métalliques solubles dans l'eau pour former les composés de cyanure de deux métaux du composant a), **caractérisé en ce que** la réaction a lieu en présence d'au moins un polyéther-siloxane du composant b).

9. Procédé selon la revendication 8, **caractérisé en ce que** le polyéther-siloxane du composant b) présente un rapport en poids entre la proportion de siloxane et la proportion de polyéther de 1 sur 1 à 1 sur 10, de préférence de 1 sur 1,2 à 1 sur 8, de manière particulièrement préférée de 1 sur 1,5 à 1 sur 5, et de manière notamment préférée de 1 sur 1,8 à 1 sur 3, par rapport à la masse molaire moyenne calculée du polyéther-siloxane.

10. Procédé selon l'une quelconque des revendications 8 ou 9, **caractérisé en ce que** des sels métalliques solubles dans l'eau du composant a1) de formule (I)
M(X)ₙ Formule (I),
avec
M choisi parmi Zn(II), Fe(II), Co(II) et Ni(II),
X anions identiques ou différents choisis dans le groupe constitué par les halogénures, les hydroxydes, les sulfates, les carbonates, les cyanates, les thiocyanates, les isocyanates, les isothiocyanates, les carboxylates, les oxalates ou les nitrates, et
n = 2 ;
sont mis en réaction avec des sels de cyanure métalliques solubles dans l'eau du composant a2) de formule (II)
(Y)ₐM'(CN)_{b}(A)_{c} (II)
avec
M' un ou plusieurs métaux choisis parmi Co(II), Co(III), Fe(II), Fe(III), Cr(III), Ir(III) et Ni(II),
Y cations de métaux alcalins ou cations de métaux alcalino-terreux identiques ou différents, de préférence identiques,
A anions identiques ou différents, choisis dans le groupe constitué par les halogénures, les hydroxydes, les sulfates, les carbonates, les cyanates, les thiocyanates, les isocyanates, les isothiocyanates, les carboxylates, les oxalates ou les nitrates,
a = 1, 2, 3 ou 4 ; b = 4, 5 ou 6 ; c = 0, 1 ou 2, les valeurs pour a, b et c étant choisies de manière à obtenir l'électroneutralité du sel de cyanure métallique ;
en présence d'au moins un polyéther-siloxane du composant b, de formule (IV)
M_{d}M'_{d1}DₑD'ₑ₁D"ₑ₂T_{f}Q_{g} (Formule IV)
dans laquelle
M = (R¹₃SiO_{1/2})
M' = (R²R¹₂SiO_{1/2})
D = (R¹₂SiO_{2/2})
D' = (R²R¹SiO_{2/2})
D" = (R⁴R¹SiO_{2/2})
T = R³SiO_{3/2})
Q = (SiO_{4/2})
d = 0 à 20 ; de préférence 1 à 10, de manière particulièrement préférée 1 à 5 et de manière notamment préférée 2 ;
d1 = 0 à 20 ; de préférence 1 à 10, de manière particulièrement préférée 0 à 2 ; de manière notamment préférée 0 ;
la somme de d et d1 étant de préférence de 2 ;
e = 0 à 300 ; de préférence 1 à 100, de manière particulièrement préférée 2 à 40, de manière notamment préférée 0 à 20 ;
e1 = 0 à 25 ; de préférence 0,1 à 15, de manière particulièrement préférée 1 à 10, de manière notamment préférée 1 à 5 ;
e2 = 0 à 10 ; de préférence 1 à 5, de manière notamment préférée 0 ;
f = 0 à 10 ; de préférence 1 à 5, de manière notamment préférée 0 ;
g = 0 à 10 ; de préférence 1 à 5, de manière notamment préférée 0 ;
à condition que
la somme de d1 et e1 soit supérieure à 0, de préférence supérieure ou égale à 1 ;
et
les R¹ sont indépendamment les uns des autres des radicaux hydrocarbonés linéaires ou ramifiés identiques ou différents de 1 à 30 atomes de carbone ou des radicaux hydrocarbonés aromatiques de 6 à 30 atomes de carbone ; les R² sont indépendamment les uns des autres des polyéthers identiques ou différents, les polyéthers pouvant comprendre des chaînes latérales, qui peuvent éventuellement également être substituées avec des hétéroatomes supplémentaires,
les R³ sont indépendamment les uns des autres des radicaux R¹ ou R² identiques ou différents,
les R⁴ sont indépendamment les uns des autres des radicaux organiques identiques ou différents de 4 à 30 atomes de carbone, à condition que R⁴ soit différent de R²,
et éventuellement en présence d'au moins un ligand complexe organique différent de b) du composant c) choisi dans le groupe constitué par les alcools, les aldéhydes, les cétones, les éthers, les esters, les amides, les urées, les nitriles, les sulfures et leurs mélanges.

11. Procédé selon l'une quelconque des revendications 8 à 10, **caractérisé en ce que** les R² dans la formule (IV) sont indépendamment les uns des autres des polyéthers identiques ou différents de formule V ou de formule VI ou de formule VII
-(O)ₕ-CH₂-CH₂-CH₂-O-(CH₂-CH₂O-)ⱼ-(CH₂-CH(R')O-)ₖ-(CH(R')-CH₂O-)_{k'}-R" (Formule V)
et
-(O)ₕ-CH₂-CH₂-O-(CH₂-CH₂O-)₃-(CH₂-CH(R')O-)ₖ-(CH(R')-CH₂O-)_{k'}-R" (Formule VI)
-(O)ₕ-(CH₂-CH₂O-)ⱼ-(CH₂-CH(R')O-)ₖ-(CH(R')-CH₂O-)ₖ'-R" (Formule VII)
avec
h = 0 ou 1,
j = 0 à 100,
k + k' = 0 à 100,
à condition que la somme de j et k soit d'au moins 3, les R' signifient indépendamment les uns des autres un groupe alkyle ou aryle éventuellement substitué de 1 à 12 atomes C, et
les R" signifient indépendamment les uns des autres un radical hydrogène ou un groupe alkyle de 1 à 4 atomes C.

12. Procédé selon la revendication 10, **caractérisé en ce que** des sels métalliques solubles dans l'eau choisis parmi le chlorure de zinc, le bromure de zinc, l'acétate de zinc, l'acétylacétonate de zinc, le benzoate de zinc, le nitrate de zinc, le sulfate de fer (II), le bromure de fer (II), le chlorure de fer (II), le chlorure de cobalt (II), le thiocyanate de cobalt (II), le chlorure de nickel (II), le nitrate de nickel (II) et leurs mélanges sont mis en réaction avec des sels de cyanure métalliques solubles dans l'eau choisis parmi l'hexacyanocobaltate de potassium (III), l'hexacyanoferrate de potassium (II), l'hexacyanoferrate de potassium (III), l'hexacyanocobaltate de calcium (III), l'hexacyanocobaltate de lithium (III) et leurs mélanges pour former les composés de cyanure de deux métaux du composant a), la réaction ayant lieu en présence d'au moins un polyéther-siloxane du composant b) et les polyéther-siloxanes du composant b) étant éventuellement des polyéther-siloxanes de formule (IV) avec d = 2, d1 = 0, e = 0 à 40, e1 = 1 à 5, e2, f et g = 0, R1 = méthyle, R2 = (Formule V), (Formule VI) et/ou (Formule VII) avec h = 0, j = 3 à 20, k = 0 à 20, de préférence 0, R' = méthyle ou éthyle et R" = hydrogène.

13. Utilisation des catalyseurs DMC selon l'une quelconque des revendications 1 à 7 ou des catalyseurs obtenus par un procédé selon l'une quelconque des revendications 8 à 12 lors de la fabrication de polymères.
